# EUROPEAN PATENT SPECIFICATION

(11) **EP 2 350 316 B1**
(45) Date of publication and mention of the grant of the patent: **16.04.2014**
(21) Application number: 09821644.3
(22) Date of filing: 19.10.2009
(51) Int. Cl.: C12Q 1/68, G01N 33/53, C12N 15/11, G01N 33/574, C12N 15/113

(54) **METHODS AND USES INVOLVING GENETIC ABERRATIONS OF NAV3 AND ABERRANT EXPRESSION OF MULTIPLE GENES**
VERFAHREN UND ANWENDUNGEN MIT GENETISCHEN ABERRATIONEN VON NAV3 UND ABERRANTER EXPRESSION MEHRERER GENE
PROCÉDÉS ET UTILISATIONS METTANT EN UVRE DES ABERRATIONS GÉNÉTIQUES DE NAV3 ET L EXPRESSION ABERRANTE DE GÈNES MULTIPLES

(30) Priority: 20.10.2008 US 196761 P
(43) Date of publication of application: 03.08.2011
(73) Proprietor: ValiPharma, London EC1N 8SS (GB)
(72) Inventor: KROHN, Kai, FI-36450 Salmentaka (FI); RANKI, Annamari, FI-00250 Helsinki (FI); CARLSSON, Emilia, FI-00270 Helsinki (FI); OVASKA, Kristian, FI-00550 Helsinki (FI); HÄYRY, Valtteri, FI-09120 Karjalohja (FI); HAUTANIEMI, Sampsa, FI-02130 Espoo (FI)
(74) Representative: Potter Clarkson LLP
(86) International application number: PCT/FI2009/050838
(87) International publication number: WO 2010/046530

(56) References cited:
- WO-A1-01/74377
- WO-A1-03/004063
- WO-A1-03/066898
- WO-A1-2008/059112
- WO-A2-2004/081190
- WO-A2-2008/048193
- CA-A1- 2 295 577
- US-A1- 2007 099 209
- O'BRYANT C ET AL: "412 POSTER An open-label study to characterize the pharmacokinetic (pk) parameters of erlotinib in patients with advanced solid tumors with adequate or moderately imparied hepatic function", EUROPEAN JOURNAL OF CANCER. SUPPLEMENT, PERGAMON, OXFORD, GB, vol. 4, no. 12, 1 November 2006 (2006-11-01), pages 126-127, XP027888871, ISSN: 1359-6349 [retrieved on 2006-11-01]
- CARLSSON E ET AL: "Expression profiling of the NAV3 protein, deleted/translocated in common forms of cutaneous T-cell lymphomas", JOURNAL OF INVESTIGATIVE DERMATOLOGY, NATURE PUBLISHING GROUP, GB, vol. 126, no. Suppl. 3, 7 September 2006 (2006-09-07), page 88, XP008112450, ISSN: 0022-202X
- LEENA KARENKO ET AL: "Primary cutaneous T-cell lymphomas show a deletion or translocation affecting NAV3, the human UNC-53 homologue", CANCER RESEARCH, AMERICAN ASSOCIATION FOR CANCER RESEARCH, US, vol. 65, no. 18, 15 September 2005 (2005-09-15), pages 8101-8110, XP002662100, ISSN: 0008-5472, DOI: 10.1158/0008-5472.CAN-04-0366
- "Agilent-014850 whole human genome microarray 4x44K G4112F (Feature Number version)", NCBI,, 17 August 2006 (2006-08-17), XP002594591,

### SEQUENCE LISTING

<110> Helsingin yliopiston rahastot
<120> Methods and uses involving genetic aberrations of NAV3 and aberrant expression of multiple genes
<130> 2081898PC
<160> 15
<170> PatentIn version 3.3
<210> 1
   <211> 23
   <212> DNA
   <213> Artificial
<220>
   <223> oligonucleotide
<400> 1
   cctgctattt tcatctttca agc 23
<210> 2
   <211> 19
   <212> DNA
   <213> Artificial
<220>
   <223> oligonucleotide
<400> 2
   ggctgggatg ctgtttgag 19
<210> 3
   <211> 30
   <212> DNA
   <213> Artificial
<220>
   <223> oligonucleotide
<400> 3
   gatgctgttt gagcgcatca tgctgggccc 30
<210> 4
   <211> 19
   <212> RNA
   <213> Artificial
<220>
   <223> oligonucleotide
<400> 4
   ggacuuaacc uauauacua 19
<210> 5
   <211> 19
   <212> RNA
   <213> Artificial
<220>
   <223> oligonucleotide
<400> 5
   gagagggucu ucagaugua 19
<210> 6
   <211> 19
   <212> RNA
   <213> Artificial
<220>
   <223> oligonucleotide
<400> 6
   cagggagccu cuaauuuaa 19
<210> 7
   <211> 19
   <212> RNA
   <213> Artificial
<220>
   <223> oligonucleotide
<400> 7
   gcuguuagcu cagauauuu 19
<210> 8
   <211> 18
   <212> DNA
   <213> Artificial
<220>
   <223> oligonucleotide
<400> 8
   atccatggag ctcagcaa 18
<210> 9
   <211> 20
   <212> DNA
   <213> Artificial
<220>
   <223> oligonucleotide
<400> 9
   ttggctgctt cttggagttt 20
<210> 10
   <211> 20
   <212> DNA
   <213> Artificial
<220>
   <223> oligonucleotide
<400> 10
   aagagcacgg ctgaagactc 20
<210> 11
   <211> 20
   <212> DNA
   <213> Artificial
<220>
   <223> oligonucleotide
<400> 11
   gcatgggttt aaaaaggcaa 20
<210> 12
   <211> 21
   <212> DNA
   <213> Artificial
<220>
   <223> oligonucleotide
<400> 12
   cgcaaaactc gctattcgac a 21
<210> 13
   <211> 19
   <212> DNA
   <213> Artificial
<220>
   <223> oligonucleotide
<400> 13
   atggcttccc tcaggcaga 19
<210> 14
   <211> 20
   <212> DNA
   <213> Artificial
<220>
   <223> oligonucleotide
<400> 14
   ttggctgctt cttggagttt 20
<210> 15
   <211> 22
   <212> DNA
   <213> Artificial
<220>
   <223> oligonucleotide
<400> 15
   caccaaatct agagctgcat ca 22

## Description

### FIELD OF THE INVENTION

The present invention relates to the fields of genetics and oncology and provides methods for detecting tumors as well as methods for predicting the prognosis to a patient.

Specifically, the present invention relates to a method of demonstrating the malignant character of a tumor or cell subpopulation in a subject and to a method of predicting a prognosis for a subject having a tumor with NAV3 copy number change and with over expression of at least one gene or gene product selected from IL23R, GnRHR and beta-catenin. The present invention also relates to uses of NAV3 gene or gene product and at least one gene and/or gene product selected from IL23R, GnRHR and beta-catenin for demonstrating the malignant character of a tumor or cell subpopulation, for predicting a prognosis to a subject having a tumor with NAV3 copy number change. Furthermore, described is a use of an antagonist, antibody or inhibitory molecule of at least one gene and/or gene product selected from specific lists for cancer therapy in a subject.

Still, described is a diagnostic kit comprising tools for detecting NAV3 copy number change in a biological sample and tools for detecting over expression of at least one gene or gene product selected from specific lists in a biological sample. The present invention also relates to a use of a NAV3 gene or gene product and at least one gene and/or gene product selected from IL23R or beta-catenin for demonstrating the malignant character of a tumor or cell subpopulation, for predicting a prognosis to a subject with a colorectal tumor, brain tumor or tumor of epidermal keratinocytes and for selecting a treatment to a subject with a colorectat tumor, brain tumor or tumor of epidermal keratinocytes.

### DESCRIPTION

Cancer progression is characterized by the development of chromosomal instability, aneuploidy, and a series of acquired genetic aberrations that affect genes important for cell growth and survival. A single gene may affect several critical pathways and contribute to the conversion of a normal cell to a cancer cell, involving a stepwise process requiring the activation of oncogenes and inactivation of tumor-suppressor genes. Recent technology has enabled the identification of even rare mutations that contribute to the development of cancer.

Colorectal cancer (CRC) development via benign precursor lesions, adenomatous polyps, along with the accumulation of genetic and epigenetic changes is one of the best-known examples of multistep carcinogenesis. Recent evidence also suggests that tissue microenvironment is of profound importance for the growth potential and spread of tumour cells (Kim B.G. et al. 2006, Nature 441: 1015-9; Reuter J.A. et al. 2009, Cancer Cell 15: 477-88) and such an effect may be driven by chronic inflammation. CRC is known to be linked to inflammatory bowel disease (IBD).

The overwhelming majority of colorectal cancers display one of the two major genomic instability phenotypes, microsatellite instability (MSI) or chromosomal instability (CIN), also called microsatellite stable (MSS). About 85% of CRC are related to MSS and exhibit aneuploidy and loss of heterozygosity (LOH), and adenomatous polyposis coli (APC) or beta-catenin mutations are the most common early molecular aberrations in this phenotype. These mutations lead to aberrant Wnt pathway activation, which is thought to initiate colon adenoma formation. Several signaling pathways are involved In the development of sporadic colon cancers. Thus, it takes several years or decades to develop colon cancer even if mutations of the adenomatous polyposis coli (APC) gene are present. Activation of KRAS is needed, too, for adenoma progression to carcinomas as a second step (Phelps R.A. et al. 2009, Cell 37: 623-34). It has also been shown that the loss of functional APC induces aneuploidy *in vivo* through transient tetraploidy (Caldwell C.M. et al. 2007, J Cell Biol 178: 1109-20), which may enhance fitness of cells containing broken or rearranged chromosomes.

We have previously shown that chromosome 12q21 aberrations, specifically allelic loss of neuron navigator 3 (*NAV3*) gene, associate with several subtypes of cutaneous T-cell lymphoma (CTCL) (Karenko L. et al. 2005, Cancer Res 65: 8101-10; Hahtola S et al. 2008, J Invest Dermatology 128: 2304-9; WO2008059112 A1) and CTCL-associated lung cancers (Hahtola S et al. 2008, Genes Chromosomes Cancer 47: 107-17). More recently, NAV3 mutations or copy number changes, respectively, have been reported in melanoma (Bleeker F. et al. 2008, Human Mutation 29: E451-59), pancreatic cancer (Bleeker F. et al. 2009, Hum Mutat 30(2): E451-9) and in human glioblastomas (Nord H. et al. 2009, Neuro Oncol Mar20). In addition, NAV3 has been found to be the only gene that was significantly differently expressed (downregulated) in adrenal carcinoma compared to adrenocortical adenomas (Soon P.H. et al. 2009, ERC). Within the cancer gene landscape, NAV3 belongs to the "hill type" candidate cancer (CAN) genes commonly mutated in human breast and colon cancer (Wood L. et al. 2007, Science 318: 1108-13).

It is considered increasingly important to concentrate research efforts on the identification of pathways affected by genetic aberrations, and thereby providing further tools for diagnosing diseases and for designing specific pharmaceuticals for individualized medicine. Now, we have found surprising and previously unknown correlations of NAV3 aberrations with specific pathways and genes related thereto. Thus, we provide novel means and methods for diagnosing or following up cancer.

Novel biomarkers or combinations of biomarkers for providing more effective and early diagnosis of potentially aggressive tumors as well as identifying tumors susceptible to targeted therapies are warranted. The present invention provides a specific solution for predicting or identifying tumor and carcinoma progression. The present invention also discloses a tool for evaluating clinical aggressiveness of tumors and patient survival.

### BRIEF DESCRIPTION OF THE INVENTION

The object of the invention is thus to provide novel methods and means for demonstrating the malignant character of a tumor and staging and monitoring a cancer, such methods and means allowing an accurate diagnosis of the disease.

Other objects of the invention are to provide novel methods and means for predicting tumor initiation or tumor progression as well as predicting a prognosis for a patient. The methods and means of the present invention are specific and reliable. The methods and means allow a well targeted therapeutic intervention, which may be life-saving.

Still another object of the invention is to provide novel biomarkers and combinations of biomarkers useful in detecting the cancer as well as in prognosis assessments.

Demonstrating NAV3 aberrations, linked to lymph node metastasis and to relevant inflammation and cell proliferation pathways, will provide a relatively simple and affordable tool to monitor cancer, specifically a cancer of colorectum, brain or epidermal keratinocytes. NAV3 regulates the expression of other genes and signaling pathways that have an effect in the malignant transformation and/or in the biological behavior of the malignant tumor. Therefore, *NAV3* affected pathways as well as different combinations thereof are also targets for the design of anti-cancer therapies.

*NAV3* copy number changes, especially NAV3 deletion, is directly involved in early carcinogenesis and opens up novel possibilities for more accurate diagnostics but also for rational therapeutic approaches.

The present invention relates to a method of demonstrating the malignant character of a tumor or cell subpopulation in a subject, the method comprising:
i) determining NAV3 copy number change in a biological sample from the subject; and
ii) determining over expression of at least one gene or gene product selected from IL23R, GnRHR, beta-catenin in the biological sample or another biological sample from the subject;
iii) demonstrating the malignant character of a tumor or cell subpopulation in a subject, when both NAV3 copy number change and over expression of at least one gene or gene product selected from IL23R, GnRHR, beta-catenin are present in the sample(s) from the subject.

A method of treating a subject having a tumor with NAV3 copy number change and with over expression of at least one gene or gene product selected from IL23R, GnRHR, beta-catenin and those listed in tables 8-12, comprising a step, wherein at least one gene or gene product with over expression is affected, is also described.

The present invention further relates to a method of predicting a prognosis comprising:
i) determining NAV3 copy number change in a biological sample from a subject;
ii) determining over expression of at least one gene or gene product selected from IL23R or beta-catenin, in the biological sample or another biological sample from the subject; and
iii) predicting a prognosis to the subject having both NAV3 copy number change and over expression of at least one gene or gene product selected from IL23R or beta-catenin in the sample(s).

Also described is a method of selecting a treatment to a subject, comprising:
i) determining NAV3 copy number change in a biological sample from the subject;
ii) determining over expression of at least one gene or gene product selected from IL23R, GnRHR, beta-catenin and those listed in tables 8-12, in the biological sample or another biological sample from the subject; and
iii) selecting a treatment to the subject having both NAV3 copy number change and over expression of at least one gene or gene product selected from IL-23R, GnRHR, beta-catenin and those listed in tables 8-12 in the sample(s).

The present invention further relates to a use of NAV3 gene or gene product and at least one gene and/or gene product selected from IL23R, GnRHR, beta-catenin for demonstrating the malignant character of a tumor or cell subpopulation with NAV3 copy number change and with over expression of at least one gene or gene product selected from IL23R, GnRHR, beta-catenin, in a subject.

The present invention further relates to a use of NAv3 gene or gene product and at least one gene and/or gene product selected from IL23R or beta-catenin for predicting a prognosis to a subject.

Furthermore described is a use of NAV3 gene or gene product and at least one gene and/or gene product selected from IL23R, GnRHR, beta-catenin and those listed in tables 8-12 for selecting a treatment to a subject.

Further described is a use of at least one gene and/or gene product selected from IL23R, GnRHR, beta-catenin and those listed in tables 8-12 for cancer therapy in a subject having a tumor with NAV3 copy number change.

Also disclosed is a use of an antagonist, antibody or inhibitory molecule of at least one gene and/or gene product selected from IL-23R, GnRHR, beta-catenin and those listed in tables 8-12 for cancer therapy in a subject having a tumor with NAV3 copy number change and with over expression of at least one gene or gene product selected from IL23R, GnRHR, beta-catenin and those listed in tables 8-12.

Furthermore disclosed is a diagnostic kit comprising tools for detecting NAV3 copy number change in a biological sample and tools for detecting over expression of at least one gene or gene product selected from IL23R, GnRHR, beta-catenin and those listed in tables 8-12 in a biological sample.

Furthermore disclosed is a use of a diagnostic kit of the invention for demonstrating the malignant character of a tumor or cell sub-population.

Furthermore disclosed is a use of a diagnostic kit of the invention for predicting a prognosis to a subject with a colorectal tumor, brain tumor or tumor of epidermal keratinocytes.

Furthermore disclosed is a use of a diagnostic kit of the invention for selecting a treatment to a subject with a colorectal tumor, brain tumor or tumor of epidermal keratinocytes.

### BRIEF DESCRIPTION OF THE DRAWINGS

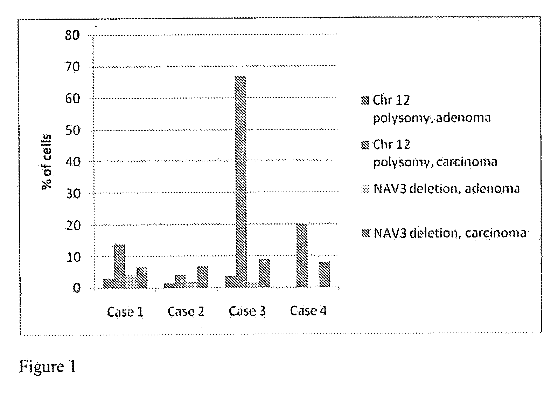

In the following the invention will be described in greater detail by means of preferred embodiments with reference to the attached drawings, in which
**Figure 1** shows amount of chromosome 12 polysomic and NAV3 deleted cells in adenoma and carcinoma samples from the same patients.

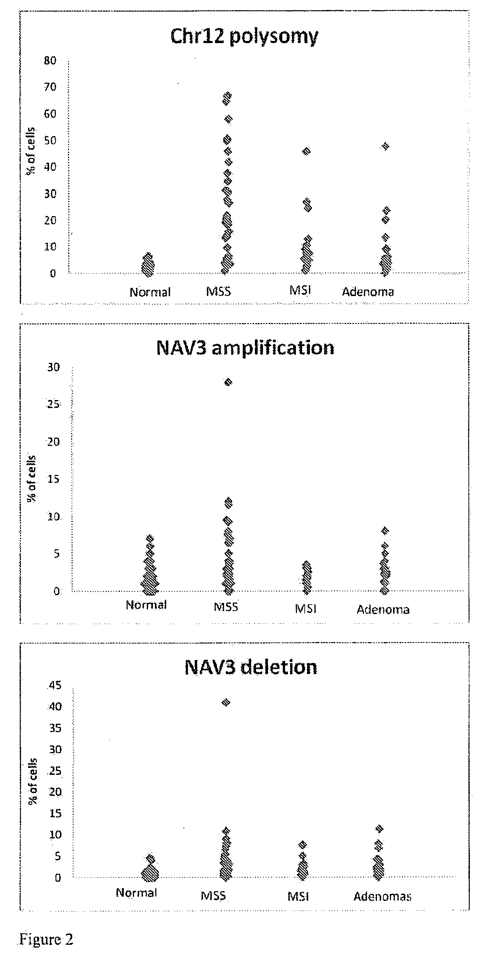

**Figure 2** shows A) Chromosome 12 polysomy, B) NAV3 amplification and C) NAV3 deletion in normal colon, MSS and MSI colon carcinoma and in colon adenoma samples. Each bullet represents one sample and 200 cells were counted from all the samples.

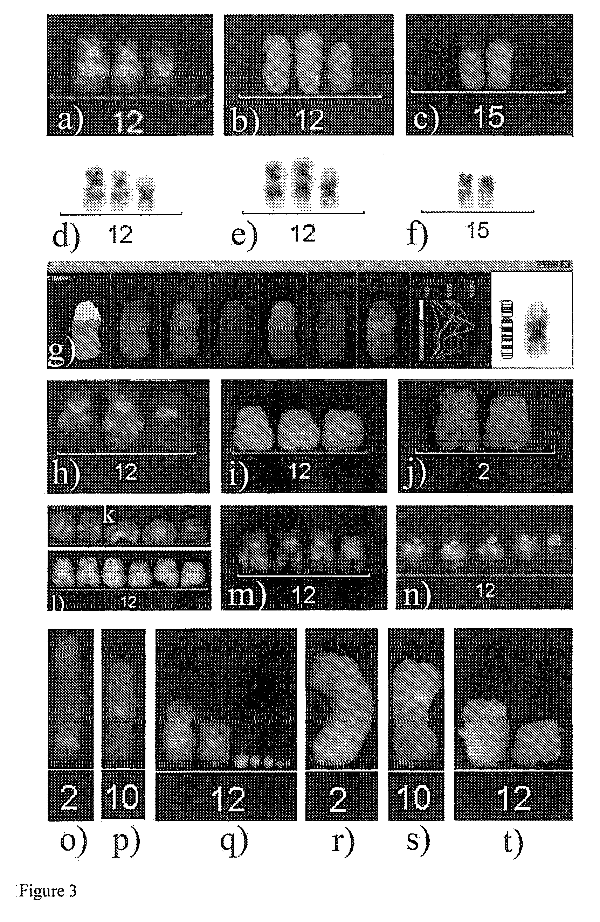

**Figure 3** shows NAV3-aberrations in colon cancer cell lines observed by BAC-probes in relation to translocations of chromosome 12 by chromosome arm specific MFISH or deletions of NAV3 in relation to deletions observed by YAC-probes. a) A metaphase of the cell line SW403 (CLL-230) showing three copies of chromosome 12 centromere (green, Alexa 488) and a missing NAV3 gene (red; BAC RP11-36P3) in one. This deletion was observed in a chromosome that received translocated material from chromosome 15q (b; arm-MFISH) and was accompanied by two full-size appearing chromosome 15 copies (c, unbalanced translocation). Respective corresponding DAPI-stainings are shown in panels d - f. By arm-specific FISH (g), the translocation from chromosome 15 (red and violet) was located to the p-arm (orange) of chromosome 12 (green and carmosine red) and the deletion was verified to q-arm. In the cell line RKO (h - j), only two of three chromosomes 12 (centromere in green) showed the NAV3-gene-specific BAC RP11-136F16 probe signals (red; h). The unbalanced translocation between chromosomes 12 (i, MFISH) and 2 (j, MFISH) abolished the NAV3-gene. T84 (CLL-248) cells showed several copies of chromosome 12 (cen in blue; k) with missing NAV3 signals in one (orange, BAC RP11-36P3). The size difference between the normal and aberrant chromosome 12 was small (I, arm-MFISH), but deletions indicated by the YAC probes 825F9 (12S326 and WI-7776, red in panel m) and 885G4 (WI-6429, WI-9760, red in panel n), extending proximally and distally of NAV3-gene, were observed. NAV3- specific signal (RP11-36P3, red) was seen in the der(2)t(2;12) (o) and in the normal chromosome 12 (q), but not in two other chromosomes 12 (p - q; centromere green) nor in the minute chromosomes with cen 12 material (q). The corresponding aberrant chromosomes commonly observed by arm-MFISH (2;12), r) der(10)t(10;12; 3),s) and i(12)(p) right, t) alongside with a normal chromosome 12, t).

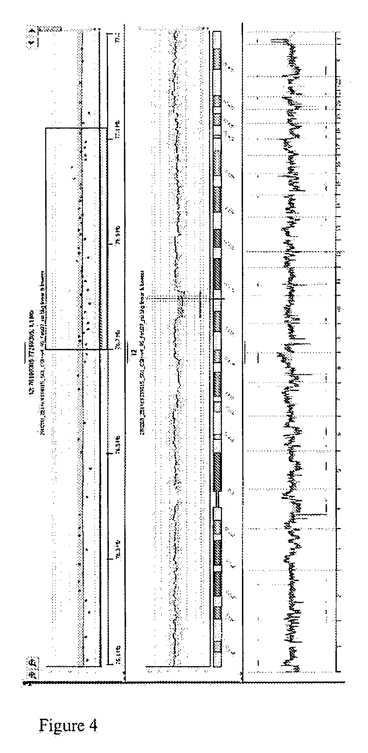

**Figure 4** shows loss of *NAV3* in a patient sample as detected by array CGH. Views from bottom: genome overview, chromosome 12, gene level where NAV3 probes marked by square box.

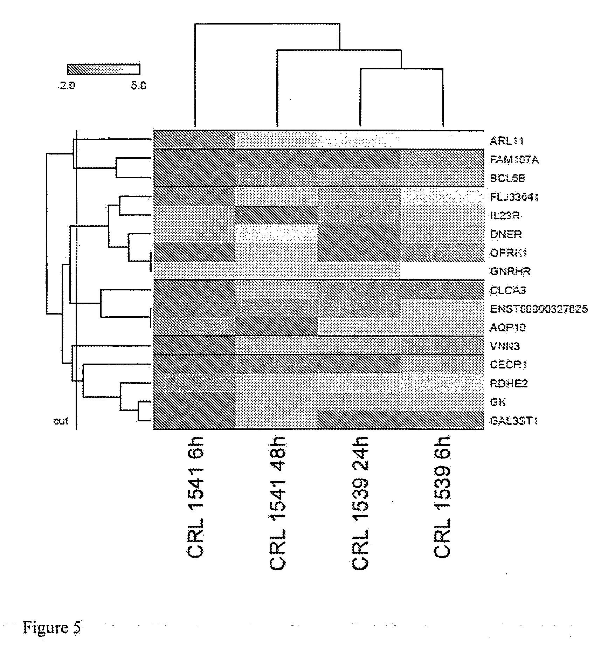

**Figure 5** shows GO clustering dendrogram and expression heat-map. The analysis is based on 16 gene products. The median number of GO annotations per gene product is 8. In addition, 23 gene products had no GO annotation and were excluded from the analysis. The colors represent expression values of the gene products. The lower expression values are indicated with red and higher expressions with white. The left dendrogram corresponds to GO based clustering, while the top dendrogram uses expression values for clustering.

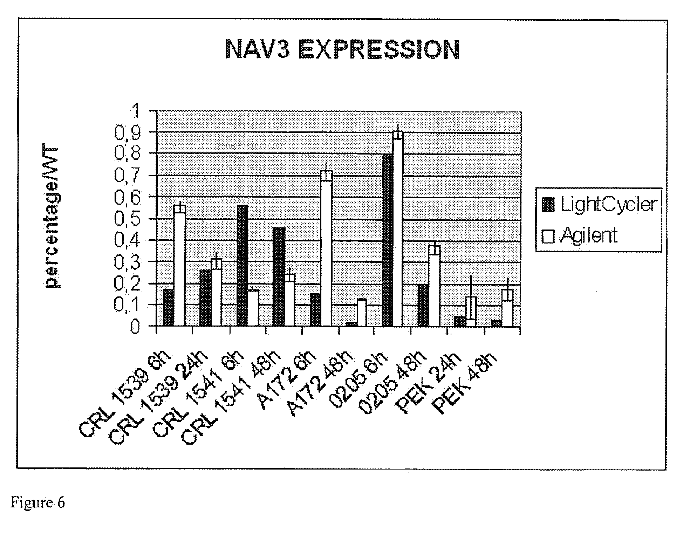

**Figure 6** shows comparison of NAV3 expression. NAV3 mRNA levels after transfection with NAV3 siRNA was assessed by LightCycler q RTPCR and Agilent 4 x 44 K microarrays.

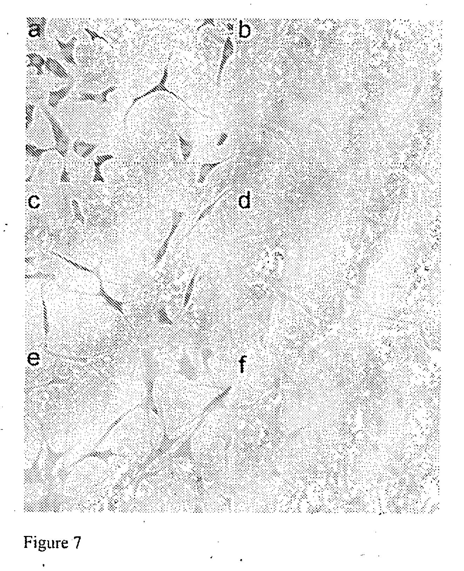

**Figure 7** shows RNA in situ hybridization of NAV3 silenced A172 glioblastoma cells. *NAV3*-RNA was detected with a probe recognizing exons 37-39 of the *NAV3* RNA (purple). *NAV3* expression in the silenced cells (e), untransfected cells (a), and cells transfected with the control RNA (c). Sense controls, with 5× more probe, for a, c, and e are shown in b, d, and f, respectively.

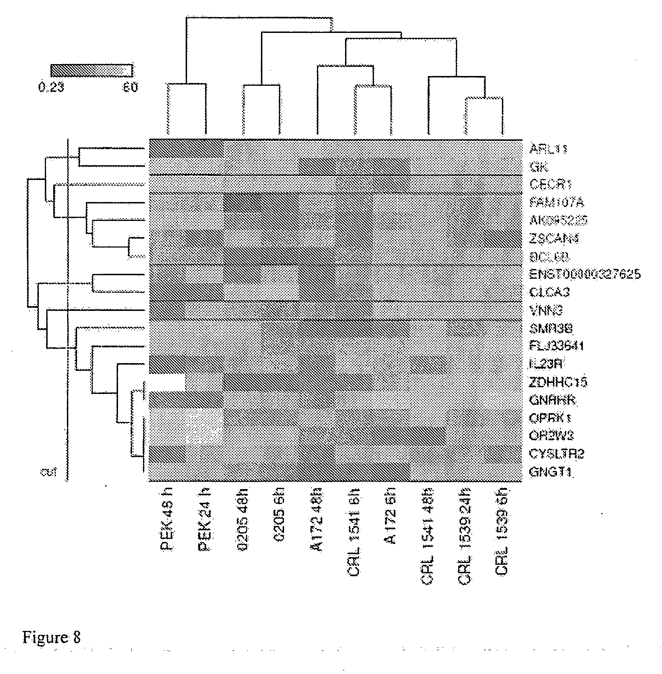

**Figure 8** shows GO clustering dendrogram and expression heat-map of the genes upregulated in eight out of ten knockdown experiments. The lower expression values are indicated with red and the higher expressions with white. GO based clustering dendrogram (left) and dendrogram clusters samples based on expression profiles (right) are shown. Genes names are on the right and each column represents a single experiment. GO groups with more than one gene are from the bottom to top: membrane (black, 9 genes), ion transport (brown, 2 genes), nuclear (green, 4 genes), and ribonucleotide binding (blue,2 genes).

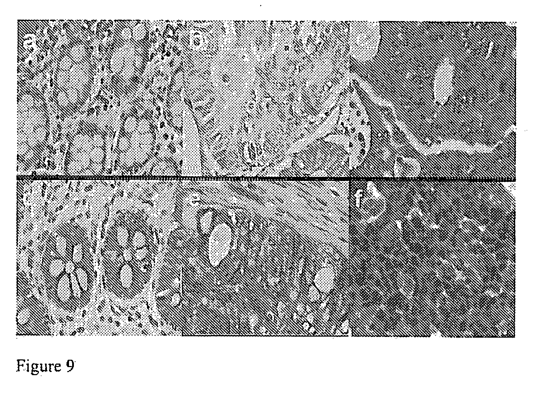

**Figure 9** shows expression and cellular localization of IL23R and beta-catenin in human colon and colorectal cancer. Representative photomicrographs of immunostaining for IL23R (a) and beta-catenin (d) in normal colon tissue. Up-regulated IL23R-immunoreactivity was observed In colorectal cancer samples with *NAV3* deletion (c, grade 3 staining, 8% *NAV3*-deleted cells) while cancers without *NAV* aberration showed no IL23R staining (b). Both samples (b and c) had 20% tumor cells with chromosome 12 polysomy. A CRC sample with no *NAV3* deletion showed normal membranous pattern of beta-catenin staining (e) while a CRC sample with *NAV3* deletion in 9% of the cells showed mainly strong nuclear localization of beta-catenin (f).

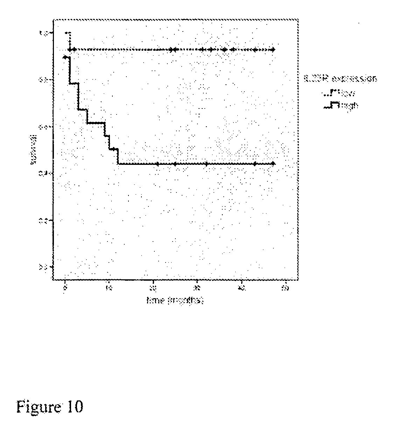

**Figure** 10 **shows** Kaplan-Meier plot of survival distributions in regard to IL23R immunoreactivity.

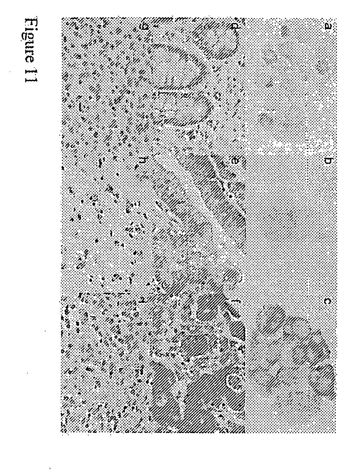

**Figure 11** shows GnRHR immunostaining of NAV3 silenced 0205 cell and colorectal and glioma tissue. Membrane staining of GnRHR in *NAV3* silenced 0205 cells (c), compared to wild type cells, (a) and to cells transfected with the control construct (b). GnRHR expression in the two colorectal cancer samples with *NAV3* deletion: patient with MSS type CRC (e), patient with MSI type CRC (f), normal colorectal tissue control (d). GnRHR staining of two glioma (astrocytoma) samples with 55% (h) and 93% (i) *NAV3* deleted cells and normal NAV3 copy number (g).

### DETAILED DESCRIPTION OF THE INVENTION

We now report that *NAV3* copy number changes are frequently found in MSS type CRC, in colon adenomas, in brain tumors, in epidermal keratinocytes and in several established cell lines. *NAV3* aberrations correlated with chromosome 12 polysomy and with lymph node involvement. Furthermore, specific siRNA gene silencing of NAV3 and expression array analyses revealed at least two important target molecules for *NAV3.*

### Diagnostic methods and methods of treatment and predicting a prognosis

All of the up-regulated genes or their gene products listed in the present application can be utilized in the development of novel diagnostic principles. In addition to determining NAV3 copy number, expression of at least one gene or gene product selected from IL23R, GnRHR and beta-catenin is studied in the methods of the present invention. As used herein the expression "gene product" refers to an mRNA, protein or to any product achieved from the gene. In a specific embodiment of the invention, the gene product is a protein.

For proteins that are secretory, methods to test their increased level in patient blood can be used as a diagnostic method. For non-secretory proteins, immunohistochemistry will be the most appropriate method to be used.

Diagnostic methods can be carried out *in vitro* or *ex vivo.* In a specific embodiment of the invention, the method is *in vitro* method.

Furthermore described is a diagnostic kit for demonstrating the malignant character of a tumor or cell subpopulation. Tools for detecting NAV3 copy number change and/or over expression of any gene or gene product may comprise suitable probes, primers or antibodies.

In a specific embodiment of the invention, the tumor is a colorectal tumor, brain tumor or tumor of epidermal ceratinocytes.

Most primary brain tumors originate from glia (gliomas) such as astrocytes (astrocytomas), oligodendrocytes (oligodendrogliomas), or ependymal cells (ependymoma). There are also mixed forms, with both an astrocytic and an oligodendroglial cell component. These are called mixed gliomas or oligoastrocytomas. Also, mixed glio-neuronal tumors (tumors displaying a neuronal, as well as a glial component, e.g. gangliogliomas, disembryoplastic neuroepithelial tumors) and tumors originating from neuronal cells (e.g. gangliocytoma, central gangliocytoma) can be encountered. Other varieties of primary brain tumors include: primitive neuroectodermal tumors (PNET, e.g. medulloblastoma, medulloepithelioma, neuroblastoma, retinoblastoma, ependymoblastoma), tumors of the pineal parenchyma (e.g. pineocytoma, pineoblastoma), ependymal cell tumors, choroid plexus tumors, neuroepithelial tumors of uncertain origin (e.g. gliomatosis cerebri, astroblastoma), etc. In a specific embodiment of the invention the brain tumor is a glioma, i.e. glioblastoma.

As used herein the expression "tumor" refers to an abnormal mass of tissue due to abnormal excess of cells divisions or lack of normal cell death.

In a specific embodiment of the invention, the tumor is a bening tumor or a malignant tumor, i.e. not cancerous tumor or cancerous tumor. The expression "adenoma" refers to a noncancerous tumor. In a specific embodiment of the invention, the tumor is a carcinoma. Carcinomas are malignant tumors derived from epithelial cells.

Methods of the present invention demonstrate the malignant character of a tumor. Malignant character refers to a malignant tumor, i.e. cancerous tumor. Malignant tumors may be aggressive i.e. fast growing and possibly metastasizing. Any "cell subpopulation", referring to a restricted population of cells, can be studied for malignant characters. The cell subpopulation may locate within the tissue-infiltrating inflammatory cells.

In the present invention, a biological sample can be any suitable tissue sample, such as biopsy from the tissue or lymph node or a metastatic tumor lesion in any body organ or whole blood. The biological sample may also be urine, stool or other body excretion. The biological sample can be, if necessary, pretreated in a suitable manner known to those skilled in the art.

The present invention also relates to a prediction of a prognosis. In a specific embodiment of the invention, a presence of a tumor with NAV3 copy number change and with over expression of at least one gene or gene product selected from IL23R or beta-catenin is associated with lymph node metastases, high grade malignancy and/or poor survival. In a specific embodiment of the invention the prognosis is poor. "Poor prognosis" refers to a high expectancy of metastasis or tumor spread, unresponsiveness to state-of-the-art treatment modalities and/or poor survival. In a patient with predicted poor survival, the life expectancy is less than in a corresponding comparative patient group.

In the clinical settings, the capability to diagnose precisely not only the type of a tumor but the genetic, functional and immunologic changes that are present in that particular tumor must be enhanced. For more detailed therapy, the choice of therapeutic molecules or antibodies can then be tailored to that particular tumor. In other words, personalized medicine is needed with a combination of at least two drugs targeting different molecules.

Furthermore described are several exploitable results that can be further used in the development of effective novel therapeutic principles and diagnostics. The finding of up-regulation of the genes, which are up-regulated in the NAV3 deficiency and are involved in malignant transformation, can be used in selecting rational therapy for a given cancer.

The decision which of the therapeutic alternatives should be used, I.e. a selection of a treatment, might be based on the testing of the malignant tumor for NAV3 copy number changes (deletion of the NAV3 gene leading to decreased expression of NAV3) and for the upregulation of any one of the genes or gene products selected from IL23R, GnRHR, beta-catenin, those listed in tables 8-12 and the IL23/IL23R as well as the GnRHR pathways.

A method of treatment further comprises a step, wherein the gene(s) or gene product(s) selected from IL23R, GnRHR, beta-catenin and those listed in tables 8-12 is affected by un-derexpressing or inactivating the gene(s) or gene product(s) or decreasing amount of the gene product(s).

A method of treatment may further comprise a step, wherein a gene or gene product of NAV3 is affected.

The gene or gene product of NAV3 is affected by overexpressing or activating the gene or gene product or increasing amount of the gene product. The gene(s) and/or gene product(s), i.e. selected from NAV3, IL23R, GnRHR, beta-catenin and those listed in tables 8-12, is activated, inactivated, overexpressed or underexpressed, or amount of the gene product is increased or decreased, at least GnRH and/or JAK/STAT signalling pathway is affected.

Any therapeutic molecule or molecules can be used for affecting the target gene products; an antagonist, antibody or inhibitory molecule is used for affecting at least one gene product. "An inhibitory molecule" refers to any molecule (e.g. small inhibitory RNA molecule or antibody), which inhibits or reduces the action of a target, e.g., the inhibitory molecule is siRNA. The up-regulation of the membrane associated proteins is of special interest, as antibody mediated therapy is presently widely used and the methods to generate humanized target specific therapeutic antibodies for target molecules are readily available.

A method of treatment may be in a form of a conventional medicament or may be given as gene therapy.

In therapy, restoration of the normal function of a gene can be used. This may be reached by enhancing the expression of functionally homologous genes, by introducing an intact gene or by using an altered form of the gene or antisense oligonucleotide against a gene in any technique presently available for gene therapy to prevent the progression of a proliferating disease. In particular, tumor cell growth may be slowed down or even stopped by such therapy. Such techniques include the *ex vivo* and *in situ* therapy methods, the former comprising transducing or transfecting an intact or altered gene (or its functional domains) in a recombinant or peptide form or as antisense oligonucleotides or in a vector to the patient, the latter comprising inserting the altered gene or oligonucleotide into a carrier, which is then introduced into the patient. Depending on the disease to be treated, a transient cure or a permanent cure may be achieved. Alternatively, monoclonal or humanized antibodies or peptides binding to a target protein can be used to suppress the function of the protein and thus tumor cell growth may be slowed down or even stopped. Antibodies against a target protein could also be used to carry other agents, such as cytotoxic substances, to the cancer cells over-expressing the gene. Such agents could then be used to kill specifically the cancer cells.

A treatment may target a gene or gene product of NAV3 and/or any gene(s) or gene product(s) listed in the present application. However, any other gene(s) or gene product(s) along the GnRH and Jak/Stat pathway may also be used as targets for therapy.

A therapeutic molecule such as an antagonist, antibody or inhibitory molecule may be administered alone or in combination with other agents or compositions. In addition to any therapeutic molecule, a pharmaceutical composition administered to a patient may also comprise any other therapeutically effective agents, pharmaceutically acceptable carriers, buffers, excipients, adjuvants, antiseptics, falling, stabilising or thickening agents, and/or any components normally found in corresponding products.

The pharmaceutical composition may be in any form, such as solid, semisolid or liquid form, suitable for administration. The administration of a medicament may for example be conducted through an oral or inhalable administration or through an intratumoral, intramuscular, intra-arterial or intravenous injection.

The subject for diagnosis, prognosis or use of the invention is a human or an animal. In a specific embodiment of the invention, the subject is a human. In a specific embodiment of the invention, the subject has a colorectal adenoma or colorectal cancer. In another specific embodiment of the invention, the subject has a colorectal tumor, brain tumor or tumor of epidermal keratinocytes.

### NAV3

*NAV3* (*neuronal navigator 3*, also called *POMFIL1*) is a spliced gene (40 exons) located in c12q21.1 and expressed in brain tissue, activated T cells, placenta, colon, and in certain cancer cell lines. The amino acid sequence of NAV3 is well conserved among different species, indicating that *NAV3* plays a fundamental role in cellular processes. Amino acid sequence homology suggests that *NAV3* participates in cell signaling and tumor suppression. NAV3 is the human homolog of unc-53 protein of *C*. *Elegans*, a critical mediator of cell migration. Also, NAV3 homologues NAV1 and NAV2 bind to intracellular filaments and regulate cell expansion and migration.

Accumulating evidence suggests that disruption of the *NAV3* gene contributes to cancer progression. The *NAV3* transcript expression is downregulated in 40% of primary neuroblastomas (Coy JF et al. 2002, Gene 290(1-2):73-94). *NAV3* is also deleted or translocated in several subtypes of cutaneous T-cell lymphoma (CTCL) (Karenko L. et al. 2005, Cancer Res 65(18):8101-10; Hahtola S. et al. 2008, J Invest Dermatol 128(9):2304-9, Vermeer M.H. et al. 2008, Cancer Res 68(8):2689-98). We have also identified *NAV3* gene copy number changes (deletions/amplifications) in cancers of epithelial origin (Hahtola S et al. 2008, J Invest Dermatol 128(9):2304-9; Hahtola S et al. 2008, Genes Chromosomes Cancer 47(2):107-17; WO 2008059112 (A1)), and allelic NAV3 deletions in colorectal cancer (Sipila L et al. 2008, EJC supplements 6(12) 118).

In a specific embodiment of the invention, NAV3 copy number change is caused by a deletion, amplification or translocation of NAV3 gene or major part of it. The copy number changes of *NAV3* gene can be determined in haploid, diploid and/or polyploid cells.

"Deletion" refers to an absence of any fragment(s) of a gene, which absence adversely affects the function of the gene. "Deletion" also refers to the absence of a whole gene or the absence of a chromosomal fragment containing the gene. In a specific embodiment of the invention, NAV3 deletion is a total or partial deletion of NAV3.

"Amplification" refers to an insertion of any fragment(s) of a gene in the presence of at least one copy of that gene. "Amplification" may also refer to the amplification of a whole gene or a chromosomal fragment containing the gene. In a specific embodiment of the invention, NAV3 amplification is a total or partial amplification of NAV3. Amplification of a gene can for example be detected by a copy number change of the gene.

"Translocation" refers to a transfer of chromosomal regions between non-homologous chromosomes. NAV3 may be translocated partly, totally or as a part of the larger chromosomal fragment comprising NAV3.

According to the method of the present invention, the presence or absence of gene copy number change can be detected from a biological sample by any known detection method suitable for detecting deletions or amplifications. Such methods are easily recognized by those skilled in the art and include fluorescence *in situ* hybridisations, such as multi-colour fluorescence *in situ* hybridisations, multi-fluor *in situ*-hybridisation (MFISH), spectral karyotyping (SKY), Combined binary ratio labelling (COBRA), colour changing karyotyping (CCK). In comparative genomic hybridization (CGH) the genetic changes are classified as DNA gains and losses. CGH reveals a characteristic pattern that includes aberrations at chromosomal and subchromosomal levels. The conventional G-banding techniques can also be used in cases were the coarse detection of gains or losses is regarded as sufficient. Preferable methods are those suitable for use in clinical laboratories.

Any known detection method suitable for detecting a gene expression of any gene or NAV3 copy number, i.e. methods based on detecting the copy number of the gene (or DNA) and/or those based on detecting the gene expression products (mRNA or protein) can be used in the methods of the present invention. Such methods are easily recognized by those skilled in the art and include conventional polymerase chain reaction (PCR)-methods, RT-PCR, *in situ* hybridisations, such as FISH, mRNA *in situ* hybridisation, Northern analysis, Southern and Western analyses, immunohistochemistry, and other immunoassays, such as ELISA. Preferable methods are those suitable for use in routine clinical laboratories.

LOH analysis may also be utilized for detecting gene copy number changes. As used herein the expression "loss of heterozygosity (LOH)" refers to the loss of a single parent's contribution to part of the cell's genome. LOH can be considered as an event to unmask a mutant allele of a gene which may play a role in suppressing tumor formation. Thus, LOH is an important marker for tumor initiation or progression. LOH in cancers can be identified by the presence of heterozygosity at a genetic locus in germline DNA and the absence of heterozygosity at the same locus in the tumor cells.

In a specific embodiment of the invention, NAV3 copy number change is confirmed by FISH, LOH, CGH, sequencing analysis, immunohistochemistry, PCR, qPCR or tissue microarray.

Markers suitable for detecting a gene expression or gene copy number changes include any biological markers such as microsatellite markers, SNP-markers, any probes, primers or antibodies associated with a target gene.

### Effects of NAV3 aberrations

Results of the present study also confirm that aberrations of NAV3 are common in various tumors and cancers. We used three different methods to look for *NAV3* copy number changes; LOH, array-CGH and FISH. With the FISH method, we observed cells with the copy number changes of chromosome 12 and the *NAV3* gene in a substantial number of cases with colorectal carcinomas (CRC). Importantly, *NAV3* deletion was also detected in 23% of adenoma samples. However, the findings in all cases showed a heterogenous population of tumour cells consisting mainly of cells with normal diploid character and abnormal cells with a variable number of chromosome 12 and/or *NAV3* label. In this setting, an allelic deletion or amplification of the gene differs from e.g. the situation with mutations in tumor suppressor genes in homogeneous tumors. It is important to note that this type of copy number aberrations can be observed only with the FISH method, which analyses individual cells, but in most cases not with array-CGH or with LOH, two methods that require that over 40% of the cells in the sample to show the same type of aberration. Our findings from an unselected clinical series of tissue samples are underscored by the fact that similar chromosome 12 polysomy and *NAV3* copy number changes were observed also in three established CRC cell lines of the MSS type and in one cell line of the MSI type.

In the cases of overt carcinoma, the cases with *NAV3* aberration clearly showed more lymph node metastases.

In the present study, in the adenomatous polyps, NAV3 loss was more often seen in cases that by classical criteria (size, differentiation) indicated higher risk for subsequent malignant transformation.

To understand the effects of *NAV3* downregulation on gene expression profiles and to characterize the effects of *NAV3* deletion *in vitro,* we silenced *NAV3* expression with RNA interference in normal colon epithelial cells, an established glioblastoma cell line, a newly derived glioblastoma cell line, and primary human keratinocytes devoid of human papilloma virus infection. Gene expression profiles at several time points after transfection were analyzed using Agilent dual-color 4 x 44K microarray. With this approach, *NAV3* gene silencing led to the consistent upregulation of 39 genes in colon cells, whereas 28 genes were upregulated in the glial cells. Among these were IL23 and GnRH receptors, and PathwayExpress analyses suggested that the GnRH and Jak-Stat signaling pathways are targeted by *NAV3*. When the results from all cell lines were combined, 49 genes, including GNRHR and IL23R were upregulated in 7 of 10 samples, at least once in each celltype. Altogether, 17 genes including the gonadotropin releasing hormone receptor (GnRHR) and interleukin 23 receptor (IL23R) were upregulated in all eight experiments with colon and glial cells.

The siRNA silencing of NAV3 mimicks the *in vivo* found allelic deletion and thus, the upregulation of the two receptor molecules, GnRHR and IL23R, known to be involved both in oncogenic processes as well as in inflammation/immune reactions, has potential importance both for the initiation of the malignant process as well as determining the biological behaviour of the malignant cells.

Importantly, in the MSS tumors, up-regulation of IL23R immunoreactivity correlated with Dukes' staging (p<0,001) and lymph node metastases (p<0.001), while nuclear beta-catenin correlated with only lymph node metastases (p=0.045). Logrank test identified up-regulated IL23R immunoreactivity as an unfavorable marker (p= 0.009).

Furthermore, *NAV3* depletion linked tissue inflammation to tumor growth by upregulating the inflammatory mediators IL23R, vanin 3, and CYSLTR2. Our findings thus suggest that *NAV3* copy number changes are directly involved in early carcinogenesis since in a microenvironment of inflammation, up-regulated IL23R gives the malignant cell a growth advantage.

### GnRHR

GnRHR is an autocrine gonadotropin hormone receptor that stimulates the secretion of luteinizing hormone (LH), follicle stimulating hormone (FSH), and gonadotropin-releasing hormone (GnRH) by pituitary gonadotrope cells (Yeung CM et al. 2005, Mol Hum Reprod 11(11):837-42. In addition to the pituitary gonadotrope cells, GnRHR is known to be expressed on the surface of lymphocytes, breast, ovary, and prostate cells. A multitude of recent reports demonstrate that the GnRH-GnRHR axis is active also in extra-pituitary cells, and upregulation of GnRHR has been observed in many cancers, especially in carcinomas (Harrison GS et al. 2004, Endocr Relat Cancer 11(4):725-48). GnRHR activation is thought to lead first to the activation of immediate early genes (IE) that affect the beta-catenin and the T-cell factor (TCF) pathways (Salisbury TB et al. 2008, Mol Endocrinol 22(6):1295-303). Beta-catenin, a member of the canonical Wnt signaling pathway, also plays an essential role in transducing the GnRH signal (Salisbury TB et al. 2008, Mol Endocrinol 22(6):1295-303) through the inactivation of glycogen synthase kinase-3, which regulates beta-catenin degradation (Gardner S et al. 2009, Neuroendocrinology 89(3):241-51). On the other hand, several reports demonstrate that the increased expression levels of beta-catenins affect lymph node metastasis of tumors (Buhmeida A et al. 2008, APMIS 116(1):1-9).

In our patient material, nuclear beta-catenin expression was surprisingly found to associate with NAV3 aberrations and to correlate with lymph node metastases. Also, our identification of GnRH as a *NAV3*-regulated target molecule represents a candidate pathway for carcinoma prevention and therapy.

### IL23R

The identification of IL-23R is linked to JAK-STAT signaling and supports our hypothesis that the pro-inflammatory tissue microenvironment affects the growth potential and spread of tumor cells. The IL-23R ligand IL-23 is secreted by activated inflammatory cells, such as macrophages and dendritic cells. The IL-23R heterodimer is composed of an IL-12 receptor beta 1 (IL-12Rbeta1) and an IL-23R (related to IL-12Rbeta2) chain (Beyer BM et al. 2008, J Mol Biol 382(4):942-55); the latter of which was upregulated in *NAV3* silenced cells. JAK2 and STAT3 both associate with IL-23R. JAK2 phosphorylates the receptor in response to IL-23 and activated STAT3 dimers translocate to the nucleus and bind to IL-23R in a ligand-dependent manner. The JAK/STAT pathway is also activated by mutations in upstream genes and has been shown to be constitutively activated in a number of human malignancies (Constantinescu SN et al. 2008, Trends Biochem Sci 33(3):122-31; Li WX. 2008, Trends Cell Biol 18(11):545-51).

Recent accumulating evidence suggests that IL-23 may redirect cytotoxic T-cell responses to tumor cells toward proinflammatory effector pathways, which nourish the tumor instead of fighting it (Langowski JL et al. 2006, Nature 442(7101):461-5). Consequently, IL-23R-expressing tumor cells are likely to gain a growth advantage and persist even in the presence of tumor specific T cells. IL-23R has also been linked to inflammatory bowel disease (IBD). In children with early Crohn's disease, mucosal levels of IL12p40 and IL23R mRNA have been shown to be significantly higher than in late disease (Kugathasan S et al.2007, Gut 56(12):1696-705). Interestingly, IBD has been associated with an increased risk of CRC and other cancers (Lakatos PL et al. 2008, World J Gastroenterol 14(25):3937-47; Hemminki K et al. 2008, Int J Cancer 123(6):1417-21).

The communication between tissue inflammatory cells and epithelial cells during cancer initiation was shown through the loss of Smad4-dependent signaling in T cells, leading to spontaneous epithelial cancers in the gastrointestinal tract of mice (Kim BG et al. 2006. Nature 441(7098):1015-9). The Smad 4 ⁻/⁻ T cells produced IL-6, which activates the JAK-STAT signaling pathway like the IL23/IL-23R complex. L-6/STAT3 signaling has also recently been shown to have a pivotal role in tumor formation in mouse models of colitis associated cancer (Grivennikov S et al. 2009, Cancer Cell 15(2):103-13).

### Other target genes

In addition to *GNRHR* and *IL23R*, five genes linked to carcinogenesis (*ARL11*, *SMR3B, FAM107A, MFSD2* and *BCLB6*) and two genes linked to inflammation (*CYSLTR2* and *VNN3*) were shown to be upregulated by *NAV3* silencing in the present study. Polymorphisms in the MYCL1 LD region, including *MFSD2,* have been recently shown to affect lung cancer survival rates although *MFSD2* genes show ethnic differences in allelic frequencies (Spinola M et al. 2007, Lung Cancer 55(3):271-7). It has been recently shown that epithelial vanin-1 controls inflammation-driven carcinogenesis in the colitis-associated colon cancer mouse model (Poyet et al, 2009), and both vanin-1 and vanin-3 expression levels are enhanced by proinflammatory cytokines in psoriasis skin cells (Jansen PA et al. 2009, J Invest Dermatol Mar 26). In this context, it is of interest to note that both psoriasis and celiac patient groups have an increased risk of cancer (Grulich AE and Vajdic CM. 2005, Pathology 37(6):409-19). The upregulation of the membrane associated protein cluster and their downstream targets are of special interest.

In the present invention, at least one gene or gene product selected from IL23R, GnRHR and beta-catenin is selected for methods, means or uses. In a specific embodiment of the invention, the gene(s) selected from IL23R, GnRHR, beta-catenin, codes for a protein, which is a membrane protein, a protein regulating cellular processes, or a purine nucleotide binding protein. Also described are methods and uses wherein the gene or gene product is selected from a group consisting of ARL11, SMR3B, FAM107A, MFSD2, BCLB6, CYSLTR2, VNN3, GNGT1, DNER, GnRHR, IL23R, beta-catenin, JAK1 and JAK3. In another specific embodiment of the invention, the gene or gene product is IL23R or/and GnRHR.

In one specific embodiment of the invention, the gene or gene product combination is at least IL23R, GNRHR and beta-catenin. In a specific embodiment of the invention, the gene or gene product combination is IL23R, GNRHR and beta-catenin.

In one specific embodiment of the invention, the gene or gene product combination is all 49 genes or gene products listed in table 9. In another specific embodiment of the invention, the gene or gene product combination is all 17 genes or gene products listed in table 8. In one specific embodiment of the invention, the gene or gene product combination is all 14 genes or gene products listed in table 10. In one specific embodiment of the invention, the gene or gene product combination is all genes or gene products listed in table 11. In one specific embodiment of the invention, the gene or gene product combination is all genes or gene products listed in table 12.

The following examples are given for further illustration of the invention. It will be obvious to a person skilled in the art that, as the technology advances, the inventive concept can be implemented in various ways. The invention and its embodiments are not limited to the examples described below but may vary within the scope of the claims.

### EXAMPLES

### Example 1. Tissue samples, cells and cell lines

### Tissue samples

### Colorectal tumor samples

Surgical biopsy samples from 59 patients (61 CRC and 10 adenoma samples), operated on CRC at Mikkeli Central Hospital were studied by FISH, LOH and immunohistochemistry. The study was approved by the Ethical Review Board of Mikkeli Central Hospital and by The National Authority for Medicolegal Affairs. Histology of the formalin-fixed paraffin-embedded tissue samples was verified by an experienced pathologist (MH) and tumours, adenomas or normal mucosa were microdissected to get pure normal or at least 50% ratio of carcinoma or adenoma tissue. Paraffin embedded sections were cut at 50 µm thickness and nuclei were isolated for FISH analysis and DNA was purified for LOH analysis following standard protocols (Hyytinen E et al. 1994, Cytometry 16: 93-96; Isola J. et al. 1994, Am. J. Pathol 145: 1301-1308). All adenomas were MSS while 14 of the 56 carcinomas had high-degree MSI.

Mononucleotide repeat markers BAT25 and BAT26 from the Bethesda panel (Boland et al., 1998, Cancer Res 58: 5248-5257), supplemented with five dinucleotide repeat markers (D12S1684, D12S326, D12S1708, D18S474, and D9S167), were used to determine the microsatellite instability (MSI) status. Samples with at least two unstable markers were considered to have MSI, whereas those with one unstable marker or none were regarded microsatellite-stable (MSS).

The following parameters were included in the statistical analyses: tumor grade, tumor stage by Dukes, and by TNM classification as defined by American Cancer Society (www.cancer.org), presence of lymph node metastases, follow-up time (months) and clinical outcome (alive, died of other reason or died of disease).

### Brain tumor samples

Samples for the FISH assay were prepared from 119 brain tumor cases. Cases consisted of 55 astrocytomas, 20 oligodendrogliomas, 13 ependymomas, 18 medulloblastomas, and 13 neuroblastomas. All tissue samples had been processed by routine formalin fixation and embedded in paraffin.

Paraffin embedded sections were cut at 50 µm thickness and nuclei were isolated for FISH analysis following standard protocols (Hyytinen E et al. 1994, Cytometry 16: 93-96; Isola J. et al. 1994, Am. J. Pathol 145: 1301-1308).

### Cells, cell lines and cell cultures

CRL-1541 and CRL-1539 normal colon cells (ATCC, Manassas, VA, USA), the glioblastoma cell line A172 derived from grade 4 giloblastoma, and primary epidermal keratinocytes (all from ATCC, Manassas, VA, USA) were grown as instructed by ATCC for no more than 30 passages. Also colorectal cancer cell lines CCL-228 (SW480), CCL-230 (SW403)., CCL-248 (T84), RKO, LIM1215, and HCA7 (ATCC, Manassas, VA, USA) were grown as instructed by ATCC. Among the CRC cell lines, RKO, LIM1215, and HCA7 are known to be mismatch repair deficient and have MSI.

Fresh cells from a grade 4 multiform gliobtastoma extracted perioperatively were used to derive the cell line 0205. Tumor tissue was mechanically dissociated, and cells were cultured in DMEM without added serum, supplemented with nutrient mixture F12 (Sigma-Aldrich, ST Louis, MO, USA), Fungizone 2,5 µg/µl (Apothecon, NJ, USA), B27 (Gibco/Invitrogen, Carlsbad, CA, USA), epidermal growth factor (EGF) 20 ng/ml(Sigma-Aldrich, ST Louis, VA, USA), basic fibroblast growth factor (bFGF) 40 ng/ml (Sigma-Aldrich, ST Louis, VA, USA), penicillin-streptomycin 100 U/ml,(Biowhittaker, Verviers, Belgium) and Glutamax (1x) (Gibco/Invitrogen, Carlsbad, CA, USA). After five passages, a clonal cell population emerged from the bulk preparation, and aliquots were frozen. For all subsequent experiments, fresh aliquots were thawed, and only cells culture less than 20 passages were used. Neuronal markers (glial fibrillary acidic protein and neuronal class beta-tubulin) expression was assessed by immunostaining. Harvesting of cells was approved by the Ethical committee of the Helsinki and Uusimaa hospital district, and written informed consent was acquired from patients.

### Example 2. FISH and LOH analysis

### FISH (fluorescence in-situ hybridization)

Cell lines CRL-1541, CRL-1539 and A172 were studied for *NAV3* copy number changes with multicolor FISH as previously described (Karenko L et al. 2005, Cancer Res 65:8101-10). FISH analysis was also performed for the tumor tissue from which the 0205 cell line was established.

Furthermore, *NAV3*-specific FISH assay was performed on nuclei isolated from patient samples (61 CRC and 10 adenoma) and on metaphase chromosomes of colon carcinoma cell lines (CCL-228, CCL-230, CCL-248, RKO, LIM1215 and HCA7). Bacterial Artificial chromosome (BAC) clones specific to *NAV3* DNA (RP11-36P3 and RP11-136F16; Research Genetics Inc., Huntsville, AL, USA) and the chromosome 12 centromere probe (pA12H8; American Type Cell Culture) were used and labelled either with Alexa 594-5-dUTP and Alexa 488-5'-dUTP (Invitrogen), respectively (patient samples) or with dioxigenin or blotin (metaphases from cell lines). The detailed methods for probe labelling, the preparation of slides for the FISH assay, and the use of arm-specific MFISH are shown below.

Also, *NAV3*-specific FISH assay was performed on nuclei isolated from samples of 119 brain tumor cases. The detailed methods for probe labelling, the preparation of slides for the FISH assay, and the use of arm-specific MFISH are shown below.

Probe labeling. For analyzing patient samples, two bacterial artificial chromosome (BAC) clones specific to *NAV3* DNA (RP11-36P3 and RP11-136F16; Research Genetics Inc., Huntsville, AL, USA) and the chromosome 12 centromere probe (pA12H8; American Type Cell Culture) were Labeled with Alexa 594-5-dUTP and Alexa 488-5-dUTP (Invitrogen), respectively, using nick translation. BAC and centromere probes were mixed together with human COT-1 DNA (Invitrogen), precipitated and diluted into hybridization buffer (15% w/v dextran sulphate, 70% formamide in 2 x SSC, pH 7.0).

For analyzing cell lines NAV3 specific BAC-probes (see above) were prepared and labeled with digoxigenin and centromere 12-specific probe was prepared and labeled with biotin or dUTP conjugated with fluorescein isothiocyanate (FITC) by nick translation as described previously (Karenko et al. 2005) or similarly with Diethylaminocoumarin - 5-dUTP (DEAC, Perkin Elmer Life and Analytical Sciences, Boston, MA, USA).

FISH methods. Nuclei slides were pretreated with 1 M sodium thiocyanate at +80°C for 5 minutes, washed three times with 2 x SSC, treated with 50% glycerol, 0.1 x SSC at +90°C for 6 minutes, washed with 2 x SSC for 3 minutes and with distilled water three times for 2 minutes. Slides were digested with proteinase K (Sigma; 8 µg/ml in 20 mM Tris-HCl, pH 7.5, 2 mM CaCl₂) at +37°C for 8 minutes. After dehydration and air drying, probe mix was added, slides were denatured for 6 min at +85°C and hybridized for 48 hr at ±37°C. Slides were washed three times with 1.5 M Urea, 0.1 x SSC at +47°C for 10 minutes, with 0.1 x SSC for 10 minutes at +47°C, three times with PBS, 0.1% NP-40 at room temperature, rinsed with distilled water, air dried and mounted in Vectashield Mounting Medium with 4',6-diamino-2 phenylindole dihydrochloride (DAPI; Vector).

FISH slides of patient samples were evaluated using Olympus BX51 microscope (Tokyo, Japan) equipped with a 60X oil immersion objective and a triple bandpass filter for simultaneous detection of Alexa488, Alexa594 and DAPI (Chroma Technology Corp., Brattleboro, VT, USA).

Conventional metaphase slides were prepared for FISH as previously described (Abdel-Rahman W. M. et al. 2001, Proc Natl Acad Sci USA 98: 2538-43). The hybridization of the probes and detection of digoxigenin-labeled NAV3-probes and biotin labeled centromere 12-specific probes were performed with avidin-FITC (Vector laboratories, Burlingame, CA, USA) and sheep anti-digoxigenin conjugated with rhodamine (Roche, Mannheim, Germany) as described previously (Karenko et al. 2005). The armspecific MFISH was performed with XaCyte kit (Metasystems GmbH, Altlusheim, Germany) as recommended by the manufacturer). The hybridized metaphases were analyzed with epifluorescence microscope (Axioplan Imaging 2, Zeiss, GmbH, Jena, Germany equipped with a CCD-camera), and MFISH-program module (Isis, Metasystems, Altlussheim, Germany) either manually or using an automatic capturing facility (Metafer, Metasystems, Altlussheim, Germany).

### Evaluation of FISH results

The FISH slides from patient-derived colorectal and brain tumor samples were analyzed, blinded to sample identity, by two independent analyzers. Results are indicated as the percentage of abnormal cells in a total number of 200 counted cell nuclei. Cells with two chromosome 12 signals (diploid cells) and only one signal for *NAV3* and cells with more than two chromosome 12 signals but with a lesser number of *NAV3* signals (e.g. three chromosome 12 centromere signals and one NAV3 signal) were interpreted as cells with *NAV3*-deletion. Cells showing more frequent *NAV3*-signals than centromere 12-signals were interpreted as cells with NAV3-amplification. A sample was considered to be *NAV3* aberrant if the percentage of interphase cells showing amplification was more than 7% or percentage of interphase cells showing deletion was more than 4% (calculated from normal distribution of normal sample results as average + 3 standard deviations). For the cell lines CCL-228, CCL-230, CCL-248, RKO, LIM1215 and HCA7 ten to 47 metaphases were analyzed for *NAV3* and centromere 12, and 6 to 11 metaphases were analyzed for arm-MFISH.

### NAV3 LOH analysis using microsatellite markers and single nucleotide primer extension, SnuPE

*NAV3* LOH assay was performed on colorectal tumor samples as previously described (Hahtola S et al. 2008, J Invest Dermatology 128: 2304-9). In addition, A/G polymorphism (rs1852464) within exon 19 of the *NAV3* gene showing up to 0.493 heterozygosity in the Caucasians/Europeans was used in the SNuPE reaction.

DNA samples were first PCR amplified using primers rs1852464F 5' CCTGCTATTTTCATCTTTCAAGC 3' (SEQ 10 NO:1) and rs1852464R 5' GGCTGGGATGCTGTTTGAG 3' (SEQ ID NO:2) to yield a 130 bp PCR fragment containing the A/G polymorphism. The PCR product was subsequently purified by Exonuclease I (10 U/µl) and SAP (Shrimp alkaline phosphatase, 2 U/µl) (ExoSAP-IT, Amersham Biosciences) and PCR Extension was performed using a fluorescently labeled extension primer 5' GATGCTGTTTGAGCGCAT-CATGCTGGGCCC 3' (SEQ ID NO:3) and nucleotide mix containing ddCTP. Extension products were 43 bp or 49 bp depending on whether G or A was present in the template and they were separated and results analyzed as previously described (Hahtola et al. 2008, J Invest Dermatology 128: 2304-9).

A sample was scored as showing LOH, if one of the alleles in the tumor sample had 40% or more decreased signal at rs1852464 or, in the event of constitutional homozygosity for this marker, at the flanking microsatellite markers compared to its matching normal (Cleton-Jansen A. M. et al. 2001, Cancer Res 61:1171-7).

### Corresponding NAV3 abnormalities are found in colorectal adenomas and carcinomas arising in the same patient when using FISH or LOH methods

Comparison of the *NAV3* copy number between the adenoma and carcinoma samples of a given patient, obtained at the same surgical operation in all but two cases, revealed that *NAV3* deletion was frequently detectable by FISH technique in the adenoma stage, too (**Figure 1**). However, the amount of *NAV3* aberrant cells was always higher in the histologically malignant lesion. Similarly, in 2 of the 3 adenomas with LOH, the matching carcinomas showed mostly similar pattern of LOH. This suggests that histologically benign adenomas having cells with *NAV3* aberrations may already have properties for malignant growth.

### NAV3 copy-number changes are found in CRC and in colorectal adenomas by using FISH or LOH methods

Cells with *NAV3* copy number changes were detected in 40% of MSS-type colorectal carcinoma samples; cells with either *NAV3* deletion or amplification were detected in 15% of samples while 15% samples showed only *NAV3* deletion and 10% had solely low level *NAV3* amplification (three to five copies). Cells with *NAV3* deletion were also detected in 12.5% of MSI-type samples and in 23% of adenoma samples. In addition, cells with chromosome 12 polysomy, most often three or five copies, were detected in 70% of MSS-type colorectal carcinoma samples, in 50% of MSI-type samples and in 31% of adenoma samples. The FISH results are illustrated in **Figure 2****.**

*NAV3* copy number changes in colon carcinoma cells were confirmed by LOH assay. LOH was detected in 21 % of MSS carcinomas and in 18% of adenoma samples. The results of each marker are shown in **Table 1.**

**Table 1. NAV3 LOH results for each marker**

| | **D12S1684** | **D12S26** | **SNuPE@rs1852464** | **D12S1708** |
|---|---|---|---|---|
| CRC, MSS | 8/37 (22%) | 10/33 (30%) | 4/23 (17%)* | 5/29 (17%) |
| CRC, MSI | 0/3 | 0/2 | 0/7* | 0/4 |
| Adenoma | 1/21 (5%) | 2/19 (11%) | 0/8* | 2/16 (13%) |

| | | | | |
|---|---|---|---|---|
| * LOH frequencies were calculated for informative cases only. SNuPE test was uninformative due to constitutional homozygosity in 5 carcinomas and in all adenomas that showed LOH by chromosome 12 microsatellites examined. | | | | |

### NAV3 copy number changes or translocations, as demonstrated with FISH, are found in established CRC cell lines

Six different established CRC cell lines (CCL-228, CCL-230, CCL-248, RKO, LIM1215 and HCA7) and two normal colon cell lines (CRL-1541 and CRL-1539) were analyzed with NAV3-specific FISH (**Table 2**). The normal colon cells CRL-1541 and CRL-1539 did not show aberrant signals for *NAV3* in relation to chromosome 12 centromere signals but in CRL-1539, 8% of the cells were tetrasomic for both of these signals. Three cell lines (SW403, RKO, T84) showed a notable portion of metaphases with loss of *NAV3* (SW-403 and T84 90% or more and RKO more than 40% of metaphases; **Table 2** and **Fig-ure 3**). The near-diploid line CCL-228 showed typically one normal chromosome 12, two abnormal chromosomes with a missing *NAV3* signal and one abnormal chromosome with *NAV3*-signal but no chromosome 12 centromere signal. A translocation of *NAV3* to another chromosome, interpreted as t(2;12) by arm MFISH, was observed in all metaphases except for one (Table 2, Figure 3).

**Table 2. Summary of CRC cell line metaphases showing numerical NAV3 or centromere 12 aberrations.**

| **Cell line** | **Type** | **The most common ploidy level, and the frequency of such metaphases/all metaphases, citromosome number range** | ***NAV3* FISH results by BAC probes** | **Arm MFISH, MFISH or YAC results** |
|---|---|---|---|---|
| CCL-230 =SW403 | CIN | near-triploid, 11/15, range 34 to 212 | Loss of *NAV3* in 14/15 metaphases | Unbalanced |
| | | | | der(12)t(12;15)(p13?:q?) |
| | | | | del(12)(q15or21) in 6/6 metaphases |
| CCL-248 =T84 | CIN | near-diploid,7/10, range 75 to 114 | Loss of *NAV3* in 9/10 metaphases | Del(12)(q?q?) in 5/5 metaphases in arm MFISH, deletion by YAC FISH in 10/10 metaphases |
| CCL-229 | CIN | near-diploid, 6/10, range 34-183 | Fragmentation or amplification of centromere 12 in 10/10 metaphases | der(2)t(2;12) (Reuter J.A. et al. 2009, Cancer Cell 15: 477-88), |
| | | | | der(10)t(3;12;10) (Chung D.C. 2000 Gastroenterology 119: 854-65), |
| | | | | i(12)(p) (Kim B.G. et al. 2006, Nature 441,1015-1019), |
| | | | | inv(12)(p12q12or13) |
| | | | | del(12)(p?)del(12)(q?) |
| | | | | (Fearon E. R. and Vogelstein B, 1990, Cell 61: 759-67) |
| RKO | MSI | near-diploid, 34/47, range 15 to 90 | Loss of *NAV3* in 21/47 metaphase studied | Unbalanced der(12)t(2;12) in 6/11 metaphases |

| | | | | |
|---|---|---|---|---|
| Cell lines LIM1215 (MIN) and HCA7 (MIN) showed as many centromere 12 as *NAV3*-signals (27/27 and 27/29 metaphases studied, respectively), as even the clonal 12q+ chromosome in HCA7 observed previously (Abdel-Rahman W.M. et al. 2001. Proc Natl Acad Sci USA 98: 2538-43) showed NAV3signal in the present study. | | | | |

### NAV3 aberrations of colorectal samples associate with chromosome 12 polysomy and with lymph node metastasis

*NAV3* aberrations of colorectal samples correlated with chromosome 12 polysomy and lymph node metastasis with statistical significance (**Table 3**). Moreover, chromosome 12 polysomy occurred more often in tumors with high grade malignancy (**Table 3**).

**Table 3. Correlations between variables of the FISH analyses, colon tumours and patient outcome among the CRC samples. The Fisher's exact test (two-sided) was used if not otherwise indicated. Survival analyses were performed using death caused by colon cancer as the primary endpoint.**

| | Chromosome 12 polysomy | Tumor grade | Dukes grade | Lymph node metastases | Patient outcome (prognosis) |
|---|---|---|---|---|---|
| NAV3 copy number change | p=0.006 | ns¹⁾ | ns | p=0.019 | Ns |
| Chromosome 12 polysomy | | p=0.019 | ns | ns | Ns |
| Nuclear betacatenin expression ²⁾ | ns | ns | ns | p<0.05 | Ns |
| Upregulated IL23R immunoreactivity ²⁾ | ns | ns | p<0.001. | p<0 001 ²⁾ | p=0.009 ³⁾ |

| | | | | | |
|---|---|---|---|---|---|
| 1) ns = not statistically significant 2) correlation analysed for the MSS patient group. 2) Chi-square test, exact, two sided 3) log-rankt test | | | | | |

### NAV3 gene copy number changes are found in brain tumors

Altogether 119 brain tumor cases of which 55 astrocytomas, 20 oligodendrogliomas, 13 ependymomas, 18 medulloblastomas, and 13 neuroblastomas were analyzed for *NAV3 gene* copy number changes and chromosome 12 polysomy using FISH (see **Table 4**). Cut-off levels for *NAV3* deletion, *NAV3* amplification, and chromosome 12 polysomy were 4%, 7%, and 10%, respectively.

Both deletion and amplification of *NAV3* were detected in astocytomas; 20% (11 out of 55) of the studied cases showed *NAV3* deletion and 11% (6 out of 55) amplification. Chromosome 12 polysomy was observed in 45% (25 out of 55) of the cases. Statistical analysis using Kaplan-Meier test showed a tendency of *NAV3* amplification predicting better outcome of the disease than deletion or normal copy number of *NAV3.*

Studied oligodendroglioma cases showed *NAV3* deletion in 15% (3 out of 20) and amplification in 30% (6 out of 20). Polysomy of chromosme 12 was detected in 43% (10 out of 23) of the cases. Chromosome 12 polysomy seemed to predict poorer outcome of the disease when analyzed with Kaplan-Meyer test.

Ependymomas showed deletion of *NAV3* in 31% (4 out of 13) of the studied cases, *NAV3* amplification in 54% (7 out of 13) and chromosome 12 polysemy in 69% (9 out of 13). Statistical analysis indicated that *NAV3* amplification and chromosome 12 polysomy predict poorer survival.

Deletion of *NAV3* was observed in 11 % (2 out of 18) of studied medulloblastoma cases, amplification of *NAV3* in 39% (7 out of 18) and polysomy of chromosome 12 in 61% (11 out of 18). Statistical analysis using Kaplan-Meier test implicated that *NAV3* deletion predict poorer survival and *NAV3* amplification, in contrast, predicted better outlook than normal copy number of *NAV3.*

No *NAV3* deletion was detected In studied neuroblastoma cases but 69% (9 out of 13) showed *NAV3* amplification and 77% (10 out of 13) chromosome 12 polysemy. In Kaplan-Meier test NAV3 amplification and chromosome 12 polysomy seemed to predict poorer survival.

**Table 4. Brain tumors were classified into five classes. Tumor cases, NAV3 aberrations (deletion, amplification), and choromosome 12 polysomy are shown as numbers and percentages of the studied cases**

| **Tumor** | ***NAV3* deletion** | ***NAV3* amplification** | **Chromosome 12 polysomy** |
|---|---|---|---|
| Astrocytoma 55/46% | 11/20% | 6/11% | 25/45% |
| Oligodenroglioma 20/17% | 3/15% | 6/30% | 10/50% |
| Ependymoma 13/11% | 4/31% | 7/54% | 9/69% |
| Neuroblastoma 13/11% | 0/0% | 9/69% | 10/77% |
| Medulloblastoma 18/15% | 2/11% | 7/39% | 11/61% |
| Altogether 119/100% | 19/17% | 35/29% | 65/55% |

Glial tumors graduses 1, 2, and 3 glial were grouped into one class and compared to gradus 4 glial tumors concerning *NAV3* aberrations (**Table 5**). Analysis showed that better differentiated lower graduses 1, 2, and 3 contained more *NAV3* amplification then gradus 4 glial tumors. *NAV3* amplification was observed in 27% (13 out of 48) of gradus 1, 2, and 3 glial tumors contrast to 5% (2 out of 38) in gradus 4 gliomas. On the contrary, gradus 4 glial tumors contained more *NAV3* deletion than graduses 1, 2, and 3. *NAV3* deletion was observed in 18% (7 out of 38) of gradus 4 glial tumors contrast to 15% (7 out of 48) in gradus 1, 2, and 3 tumors.

**Table 5. Glial tumor graduses 1, 2, and 3 were grouped into one class and gradus 4 glial tumors formed the other class. Tumor cases, NAV3 amplification, and NAV3 deletion are shown as numbers and percentages of the studied cases in these two classes.**

| | ***NAV3* deletion** | ***NAV3* amplification** |
|---|---|---|
| **Gradus 1, 2, and 3 glial tumors** 48 / 56% | 7/15% | 13/27% |
| **Gradus 4 glial tumors** 38/44% | 7/18% | 2/5% |
| **Altogether** 86/100% | 14/16% | 15/17% |

### Example 3. CGH

### Array CGH

DNA was extracted from 50 micrometer paraffin embedded tissue sections by standard protocol. Reference-DNA was extracted from blood pooled at the Finnish Red Cross from 4 healthy males and females. DNA was then digested, labeled and hybridized to a 244K oligonucleotide array according to the manufacturer's (Agilent Technologies, Santa Clara, USA) protocol. Samples were scanned with a DNA microarray scanner and analyzed using Feature Extraction and CGH Analytics software (Agilent Technologies, Santa Clara, USA). Analysis was performed using the z-score and a 1Mb moving average window. Log2-values under +/- 0.4 were not considered aberrant. Three colon carcinoma cell-lines and two colon carcinoma tumour samples were analyzed using this method.

### Array-CGH analysis of selected cases of patient material and of CRC cell lines

Array-CGH studies were performed on two samples from the patient material and on three established CRC cell lines CLL-230, CLL-248 and CLL-228. Array-CGH data demonstrated a deletion in 12q21 spanning the *NAV3* locus in one of the patient samples, thus confirming the FISH results (this patient sample had 41% *NAV3* deleted cells by the FISH method) (**Figure 4**). However, the other patient sample showed normal results in this analysis, probably due to an insufficient proportional number of *NAV3* aberrant cells in the sample (28% of cells showing amplified *NAV3* signals in FISH analysis). Array-CGH analysis of colon carcinoma cell lines showing *NAV3 loss* in FISH revealed major alterations in chromosome 12 as well as in other chromosomes, as is to be expected in cultured cancer cells. In the CLL-230 line, a wide deletion spanning the *NAV3* locus was detected in 12q. This deletion was not detected in the other (two) cell lines (CLL-248 and CLL-228) which in turn demonstrated amplifications of the other parts of the chromosome.

### Example 4. RNAi and gene expression microarrays

### METHODS

### NAV3- gene silencing in vitro

The *NAV3*-gene was silenced with pooled siRNA oligos (On-Target SMART pool, Dharmacon, Chicago, IL, USA) The specific sequences of the oligos were as follows: 5'-GGACUUAACGUAUAUACUA-3' (SEQ ID NO:4) (Exon 12), 5'-GAGAGGGUCUUCAGAUGUA-3' (SEQ ID NO:5) (Exon 38-39), 5'-CAGGGAGCCUCUAAUUUAA-3' (SEQ ID NO:6) (Exon 7), and 5'-GCUGUUAGCUCAGAUAUUU-3' (SEQ ID NO:7) (Exon 30-31).

Glioblastoma cells A172, the primary epidermal keratinocytes, and the normal colon cell lines CRL-1541 and CRL-1539 were cultured in 6-well plates to 70% confluence, and thereafter transfected with 200 pmol of *NAV3* siRNA pool or scrambled control siRNA (Dharmacon, IL, USA), using Dharmafect1 transfection reagent (Dharmacon). Glioblastoma cells 0205 were induced to grow as an adherent monolayer by altering the above mentioned culture conditions as follows: bFGF and EGF were withdrawn and the medium was supplemented with 10% FCS for 4 days before transfection. For the transfection of one 6-plate well, the siRNA was diluted to 1 µM in 100 µl of 1 x siRNA buffer (received from manufacturer) and mixed with an equal amount of normal growth medium. Four microliters of transfection reagent was incubated for 5 minutes at room temperature with 196 µl of growth media. The siRNA dilution was added and incubated for 20 minutes at room temperature before addition to the cells in 1600 µl of growth media. Six hours post transfection, the transfection medium was replaced with normal growth medium. The viabiiity of the cells was monitored 48 hours after transfection with trypan blue staining. All cell types showed over 70% viability, and in addition, only adherent cells were used for RNA preparation.

### Gene expression microarrays

To identify genes regulated by *NAV3*, changes in gene expression profiles induced by *NAV3*-targeted RNAi were measured with Agilent 4 x 44K dual-color microrrays and oligonucleotide probes (Biochip center, Biomedicum Helsinki, http://www.helsinki.fi/biochipcenter/). For this analysis, total RNA samples from the siRNA-transfected cells were obtained 6, 24, and 48 hours post-transfection (CRL 1541: 6 and 48 h, CRL 1539: 6 and 24 h, A172: 6 and 48 h, 0205: 6 and 48 h, primary epidermal keratinocytes (PEK): 24 and 48h).

Cells were lysed in 350 µl of RLT buffer (Qiagen), RNA was purified with the RNeasy Micro or Mini kit (depending on the amount of cells) (Qiagen, Hilden, Germany) and stored at -70°C. The total RNA obtained from the *NAV3-*silenced cells was hybridized with the corresponding time point samples from the same cells transfected with scrambled oligonucleotides. Before hybridization, the quality of the RNA samples was assessed with the 2100 Bioanalyzer (Agilent Technologies).

### Microarray analysis

Microarray data from the two normal colon cell lines CRL-1541 and CRL-1539, and data from glioblastoma cells and primary epidermal keratinocytes transfected with the NAV3 silencing oligos or with the control oligos were analyzed. Probe intensities were background corrected and normalized with LOWESS using Agilent Feature Extractor 9.1.3.1. For each sample, fold changes within each sample were computed, and 200 probes with the highest expression and 200 probes with the lowest expression were considered as differentially expressed. Genes that were consistently differential expressed in all normal colon cell lines and tumor samples were used for computational pathway analysis using PathwayExpress (Draghici S. et al. 2007, Genome Res 17(10): 1537-45). In this method, expression values of differentially expressed genes are used to compute an impact factor and an associated p-value for signalling pathways in the KEGG database [REFE: PMID: 18477636], taking into account the topology of the pathways.

With this method, a gene coding a protein located upstream of a pathway has more numerical impact than downstream proteins.

In addition, a novel Gene Ontology (GO) based clustering method was used to reveal gene clusters that share a common biological function or location in the cell (Ovaska K et al. 2008, BioData Min 1(1):11). Distances between genes are computed using the Lin semantic similarity measure (Lin. Proceedings of the 15^{th} International Conference on Machine Learning 1998: 296-304) based on GO annotations. Genes having similar GO annotations have shorter distances from each other and are clustered closely. The clusters, together with expression values, are visualized using a heat map and dendrograms.

### qRT-PCR

Efficient *NAV3*-silencing and the upregulation of IL23R and GnRHR were confirmed by Light Cycler qRT-PCR and RNA in situ hybridization.

For the quantitative RT-PCR, total RNA was reverse transcribed with Revert Aid^{™} First Strand cDNA Synthesis Kit (Fermentas, St.Leon-Rot, Germany) according to manufacturer's instructions. To prepare standard curves, serial dilutions of cDNA from A172 cells were made. The reactions were performed with a LightCyder480^{™} apparatus using the LC 480 SYBR Green 1 master kit (Roche Diagnostics, Mannheim, Germany). Briefly, the DNA was denatured in 95°C for 5 minutes, then 45 cycles of 95°C, 58°C, and 72°C were run for 10, 15, and 10 seconds respectively, followed by melting curve analysis according to the manufacturer's guidelines. After background adjustment, the fit point method was used to determine the crossing-point value as previously described (Linja MJ et al. 2004, Clin Cancer Res 10(3):1032-40). For the normalization of the expression levels, the expression of the TATA-binding protein (TBP) was measured as described (Linja MJ et al. 2004, Clin Cancer Res 10(3):1032-40). The relative expression level was obtained by dividing the values for *NAV3* with the TBP value. In addition to the melting curve analysis, the PCR products were separated by 1.5% agarose gel electrophoresis to ensure the right product size. The upregulation of IL23R and GnRHR was also confirmed by qRT PCR, increasing the annealing temperature to 60°C. All primers used in the reactions are listed in **Table 6**.

**Table 6. PCR primers used in qRT-PCR**

| Gene | fwd primer | rew primer |
|---|---|---|
| NAV3 | ATCCATGGAGCTCAGCAA (SEQ ID NO:8) | TTGGCTOCTTCTTGGAGTTT (SEQ ID NO:9) |
| GnRHR | AAGAGCACGGCTGAAGACTC (SEQ ID NO:10) | GCATGGGTTTAAAAAGGCAA (SEQ ID NO:11) |
| IL23R | CGCAAAACTCGCTATTCGACA (SEQ ID NO:12) | ATGGCTTCCCTCAGGCAGA (SEQ ID NO:13) |

### NAV3 RNA in situ hybridization of NAV3-silenced cells

To assess knockdown efficiency of the *NAV3* gene, we performed NAV3-targeted RNA *in situ* hybridization of the siRNA-transfected cells of cell lines A172 and 0205. The expression of *NAV3* was compared to untransfected cells and cells transfected with the scrambled oligos. Briefly, *NAV3* mRNA transcripts were detected with a probe recognizing exons 37-39, with overnight hybridization at 45°C.

### mRNA in situ hybridization for NA V3 mRNA

### Preparation of RNA probes

The RNA probes were generated based on specific exon locations of *NAV3* gene exerting following oligonucleotides (exon 37-39: antisense 5'-TTGGCTGCTTCTTGGAGTTT-3' (SEQ ID NO: 14), sense 5'-CACCAAATCTAGAGCTGCATCA-3' (SEQ ID NO: 15)), The resulting PCR products were cloned in pCR®II-TOPO® vector (Invitrogen). RNA probes were labeled using the DIG RNA labelling Kit (SP6/T7, Roche) according to the manufacturer's instructions.

### In situ hybridization for fixed cells

The cell lines A172 and 0205 were cultured on sterile glass slides (LAB-TEK II chamber Slide w/Cover RS Glass Slide, Nalge Nunc International) and fixed in 4% paraformaldehyde (MERCK) - PBS for 15 min at room temperature. The slides were rinsed with PBS and refixed in 1% PFA-PBS (7 min, +4 - 8°C) followed by rewashing in PBS. Slides were acetylated in 0.1 M triethanolamine (TEA, Riedel-de Haan) pH 8.0 - 0.25% acetic anhydride (MERCK) and prehybridized with 4 x SSC-50% deionized formamide. In situ hybridization was performed in a humidified chamber overnight at 45°C using hybridization solution containing denatured DIG-RNA antisense probe (4 ng/µl) or sense probe (19 ng/µl). The posthybridization slides were washed with prewarmed buffers at 37°C water bath (1x10min, 1x5min 2 x SSC, and 2 x 5min 1 x SSC) and the excess of RNA was removed with RNase (20 µg/ml RNase A, Sigma; 500 mM NaCl; 10 mM Tris pH 8.0; 1 mM EDTA) for 30 min at 37°C). After being washed, the hybridized probes were detected immunologically by anti-DIG-alkaline phosphatase Fab diluted 1:1000 in blocking solution. The slides were washed and incubated overnight in humidified chamber with color substrate solution (1:50 NBT/BCIP Stock Solution (Roche) in detection buffer containing 0.002 mM fresh levamisole (Vector Laboratories)). To terminate the reaction slides were washed, rinsed briefly in water and counterstained with 0.1% Kemchrot (1 x 5min, Gurr, BDH Chemicals). All reagents and instruments were either treated with diethyl pyrocarbonate (DEPC) or were RNase free.

### RESULTS

### NAV3 gene silencing resulted in upregulation of GnRHR and lL23R expression

### Normal colon cell lines

To identify *in vivo* relevant target genes of NAV3, we studied the gene expression profiles of *NAV3*-silenced normal colon cells CRL-1541 and CRL-1539 (with normal NAV gene copy numbers). In these experiments we identified, among 39 genes constantly upregulated in the NAV3-silenced normal colon cells, the upregulation of two key receptors involved in carcinogenesis. Firstly, the gonadotropin releasing hormone receptor (GnRHR) pathway was identified using PathwayExpress to be statistically significantly enriched (multiple hypothesis corrected p-value <0.001), and this result was driven by the upregulatlon of *GNRHR,* transcript variant 1, with fold changes 4.7 - 32.4. Secondly, the Jak-STAT pathway was identified to be marginally statistically significant (corrected p-value <0.058) and was affected by upregulation of interleukin 23 receptor (IL23R), with fold changes 3.4-14.01. The upregulation of these receptors was confirmed by qRT-PCR in selected samples. See **Figure 5****.**

### Cell lines and tissue samples

With multicolor FISH, the number of *NAV3* signals was compared to the number of centromere 12 signals, and cells thereafter defined to have either normal, amplified, or deleted *NAV3* signals. The studied cell lines CRL-1541, CRL-1539, 0205 and A172 were shown to consist mainly of cells without *NAV3* aberrations (**Table 7**) and were therefore suitable for *NAV3* silencing studies. The efficient silencing of *NAV3* was confirmed by qRT-PCR and RNA *In situ* hybridization (**Figures 6** and **7**). Also, both probes for *NAV3* present on the Agilent 4 x 44k microarray were under-expressed with a median fold change of 0.26, indicating that *NAV3* silencing was effective. All methods showed coinsistent downregulation of *NAV3* in all cell types at all studied time points.

**Table 7. NAV3 and chromosome 12 centromere FISH**

| Cell line or tissue sample | Cell type | Number of nuclei analyzed | % of nuclei with normal signals | % nuclei with NAV3 polysomy | % of nuclei with amplified NAV3 signal | % of nuclei with deleted NAV3 signal |
|---|---|---|---|---|---|---|
| CRL-1541 | Colon | 30 | 100 | 0 | 0 | 0 |
| CRL-1539 | Colon | 37 | 91.9 | 8.1 | 0 | 0 |
| 0205 | Glioblastoma | 200 | 98.5 | 0 | 0.5 | 0.5 |
| A172 | Glioblastoma | 108 | 11.1 | 74 | 5.6 | 9.3 |
| 0205 tumor | Glioblastoma | 206 | 97.09 | 0.49 | 1.46 | 0.49 |

When the gene expression profiles of all *NAV3*-silenced CRL-1541 and CRL-1539 normal colon cells, the glioblastoma cell line A172 derived from grade 4 glioblastoma, and primary epidermal keratinocytes (all from ATCC, Manassas, VA, USA) were evaluated, we identified 17 genes upregulated in all experiments (**Table 8**). Of these, the upregulation of two key receptors involved in carcinogenesis were noted. First, the gonadotropin releasing hormone receptor (GnRHR) pathway was identified using PathwayExpress to be statistically significantly enriched (multiple hypothesis corrected p-value <0.001). This result was driven by the upregulation of *GNRHR* transcript variant 1 which had a median fold change of 13.7. Second, the Jak-STAT pathway was identified to be marginally statistically significantly altered (corrected p-value <0.058) by upregulation of the interleukin 23 receptor (IL23R), which had a median fold change of 7.2. The upregulation of these receptors was confirmed by qRT-PCR in selected samples. We also decided to include the gene expression profiles of *NAV3*-silenced primary human keratinocytes (PEK) in the analysis. We then identified 52 genes, including *GnRHR* and *IL23R* that were upregulated in 7 of the 10 experiments performed with all three cell types (**Table 9**).

**Table 8. List of 17 genes upregulated in all eight NAV3-silencing experiments with glial and colon cells.**

| **Abbreviation** | **Max fold change** | **Min fold change** | **Full Name** |
|---|---|---|---|
| C12orf42 | 47.8 | 12.7 | Homo sapiens chromosome 12 open reading frame 42 (C12orf42), inRNA [NM 198521] |
| GNRHR | 32.4 | 47 | Homo sapiens gonadotropin-releasing hormone receptor (GNRHR), transcript variant 1 mRHA [NM 000406] |
| ARL11 | 30.2 | 6.8 | Homo sapiens ADP-ribosylation factor like 11 (ARL11), mRNA [NM 138450] |
| AF334588 | 31.8 | 12.8 | Homo sapiens P25 mRNA complete cds, [AF334588] |
| A 24 P799680 | 29.3 | 6.1 | Unknown |
| UNQ6490 | 41.1 | 5.5 | Homo sapiens clone DNA147309 YPLR6490 (UNQ6490) mRNA, complete cds, [AY358209] |
| FLJ33641 | 23.4 | 59 | Homo sapiens hypothetical protein FLJ33641 (FLJ33641), mRNA [NM_152687] |
| CN480368 | 25,3 | 3.8 | UI-H-EU0-azt-m-22-0-UI,s1 NCI_CGAP_Carl Homo sapiens cDNA clone UI-H-EU0-azt-m-22-0-UI 3' mRNA sequence [CN480368] |
| NP297856 | 20.0 | 40 | GB\|AF132199,1\|AAG35545,1 PRO1460 [NP297856] |
| ENST00000329949 | 23.6 | 49 | Homo sapiens POM121-like protein, mRNA (cDNA clone IMAGE 40053742), [BC112340] |
| ENST00000360623 | 17.2 | 5.5 | Homo sapiens clone F22H myosin-reactive immunoglobulin heavy chain variable region mRNA, partial cds, [AF035042] |
| THC2407334 | 17.0 | 4.2 | Unknown |
| IL23R | 14.0 | 34 | Homo sapiens interleukin 23 receptor (IL23R), mRNA [NM 144701] |
| THC2443880 | 13.3 | 42 | Unknown |
| VNN3 | 12.4 | 4.5 5 | Homo sapiens vanin 3 (VNN3), transcript variant 1, mRNA [NM 018399] |
| LOC348174 | 50.2 | 7.9 | Homo sapiens cDNA FLJ38732 fis, clone KIDNE2010750, [AK096051] |
| ENST00000372121 | 11.2 | 3.9 | Homo sapiens hypothetical gene supported by BC006119, mRNA (cDNA clone IMAGE:3505629), partial cds [BC006119] |

**Table 9. List of 49 genes upregulated in at least 7/10 silencing experiments and once in each cell type. In the right column, fold changes of gene expressions in cells compared to the gene expressions of the same genes in the siRNA silenced cells are pointed out.**

| Gene Name | Description | Median fold-change |
|---|---|---|
| AK021467 | Homo sapiens cDNA FLJ11405 fis clone HEMBA1000769 [AK021467] | 24.5 |
| C12orf42 | Homo sapiens chromosome 12 open reading frame 42 (C12orf42) mRNA [NM_198521] | 24.1 |
| AF334588 | Homo sapiens P25 mRNA complete cds [AF334588] | 23.2 |
| A 24 P799680 | Unknown | 18.9 |
| CN480368 | UI-H-EU0-azi-m-22-0-UI.sl NCI_CGAP_Carl Homo sapiens cDNA clone UI-H-EU0-azi-m-22-0-UI 3', mRNA sequence [CN480368] | 14.3 |
| GNRHR | Homo sapiens gonadotropin releasing hormone receptor (GNRHR) transcript variant 1, mRNA [NM 000406] | 13.7 |
| LOC348174 | Homo sapiens cDNA FLJ38732 fis, clone KIDNE2010750. [AK096051] | 13.7 |
| THC2378839 | CR457228 FLJ20225 {Homo sapiens;} , partial (19%) [THC2318839] | 13.2 |
| ENST00000360623 | Homo sapiens clone F22H myosin-reactive immunoglobulin heavy chain variable region mRNA partial cds. [AF035042] | 12.4 |
| UNQ6490 | Homo sapiens clone DNA 147309 YPLR6490 (UNQ6490) mRNA, complete cds. [AY358209] | 12.4 |
| CECR1 | Homo sapiens cat eye syndrome chromosome region, candidate 1 (CECR1), transcript variant 1, mRNA [NM 017424] | 11.7 |
| OR2W3 | Homo sapiens olfactory receptor, family 2, subfamily W, member 3 (OR2W3), mRNA [NM_001001957] | 11.3 |
| A 32 P93894 | Unknown | 11.2 |
| GK | Homo sapiens glycerol kinase (GK), transcript variant 1, mRNA [NM_203391] | 10.0 |
| THC2407334 | Unknown | 9.9 |
| ARL11 | Homo sapiens ADP-ribosylation factor-like 11 (ARLI1), mRNA [NM_138450] | 9.9 |
| AA903523 | AA9C3523 ok50h02.s1 NCI CGAP Lei2 Homo sapiens cDNA clone IMAGE: 1517427 3', mRNA sequence [AA903523] | 9.8 |
| TPD52L3 | Homo sapiens tumor protein D52-like 3 (TPD52L3), transcript variant 1, mRNA [NM_033516] | 9.7 |
| FLJ33641 | Homo sapiens hypothetical protein FLJ33641 (FLJ33641), mRNA [NM_1526871 | 9.5 |
| A 24 P929289 | Unknown | 8.5 |
| ZDHHC15 | Homo sapiens zinc finger, DHHC-type containing 15 (ZDHHC15), mRNA [NM_144969] | 8.3 |
| ENST00000329949 | Homo sapiens POM121-like protein, mRNA (cDNA clone IMAGE 40053742), [BC112340] | 8.0 |
| ANKRD45 | Homo sapiens ankyrin repeat domain 45 (ANKRD45) mRNA [NM_198493] | 7.8 |
| THC2443880 | Unknown | 7.5 |
| CLCA3 | Homo sapiens chloride channel, calcium activated family member 3 (CLCA3), mRNA [NM_004921] | 7.5 |
| ENST00000327625 | Unknown | 7.2 |
| IL23R | Homo sapiens interleukin 23 receptor (IL23R), mRNA [NM_144701] | 7.2 |
| VNN3 | Homo sapiens vanin 3 (VNN3) transcript variant 1, mRNA [NM_018399] | 7.0 |
| OPRK1 | Homo sapiens opioid receptor kappa 1 (OPRK1), mRNA [NM_000912] | 6.9 |
| BX113452 | BX113452 Soares infant brain 1N1B Homo sapiens cDNA clone IMAGp998L21165 mRNA sequence [BX113452] | 6.7 |
| ENST00000372127 | Homo sapiens hypothetical gene supported by BC006119, mRNA (cDNA clone IMAGE 3505629), partial cds, [BC006119] | 6.6 |
| CYSLTR2 | Homo sapiens cysteinyl leukotrine receptor 2 (CYSLTR2), mRNA [NM_020377] | 6.5 |
| SMR3B | Homo sapiens submaxillary gland androgen regulated protein 3 homolog B (mouse) (SMR3B), mRNA [NM_006685] | 6.3 |
| THC2319152 | Unknown | 6.3 |
| THC2405170 | Unknown | 6.2 |
| U22172 | Human DNA damage repair and recombination protein RAD52 pseudogene mRNA, partial cds. [U22172] | 6.1 |
| GNGT1 | Homo sapiens guanine nucleotide binding-protein (G protein), gamma transducing activity polypeptide 1 (GNGT1), mRNA [NM_021955] | 5.9 |
| AF078533 | Homo sapiens evolutionarily related interleukin-1beta converting enzyme mRNA complete cds [AF078533] | 5.8 |
| FAM107A | Homo sapiens family with sequence similarity 107, member A (FAM107A), mRNA [NM_007177] | 5.7 |
| THC2314833 | Unknown | 5.7 |
| ZDHHC15 | Homo sapiens zinc finger BHHC-type containing 15 (ZDHHC15) mRNA [NM_144969] | 8.3 |
| CCDC62 | Homo sapiens coiled-coil domain containing 62 (CCDC62), transcript vanant 1, RNA [NM_032573] | 5.7 |
| THC2351769 | Unknown | 5.7 |
| AK095225 | Homo sapiens cDNA FLJ37906 fis, clone COLON20043318, [AK095225] | 5.3 |
| A 23 P134405 | Unknown | 5.3 |
| BCL6B | Homo sapiens B-cell CLL/lymphoma 6, member B (zinc finger protein) (BCL6B), mRNA [NM_181844] | 5.2 |
| NP297856 | GB[AF132199.1] AAG35545.1 PRO1460 [NP297856] | 5.1 |
| ZSCAN4 | Homo sapiens zinc finger and SCAN domain containing 4 (ZSCAN4) mRNA [NM_152677] | 5.1 |
| THC2438003 | Q9BVX4 (Q9BVX4) MGC5566 protien, partial (23%) [THC2438003] | 4.8 |
| AF119887 | Homo sapiens PRO2610 mRNA, complete cds [AF119887] | 4.8 |
| X92185 | H.sapiens mRNA for alu elements [X92185] | 4.7 |
| THC2455392 | Unknown | 4.3 |
| C19orf30 | Homo sapiens chromosome 19 open reading frame 30 (C19orf30), mRNA [NM_174947] | 4.0 |

### Gene Ontology analysis of NAV3 regulated genes

Gene ontology based clustering was performed to identify biological processes affected by *NAV3* depletion. To increase the number of genes for GO analysis, we performed clustering on the 52 genes that were upregulated in 7 of 10 experiments. From this group, we identified four GO clusters (Figure 8): membrane (nine genes), ion transport (two genes), nuclear (four genes), and ribonucleotide binding (two genes). The ribonucleotide binding cluster included two genes: glycerol kinase and *ARL11*. Glycerol kinase is a key enzyme in the regulation of glycerol uptake and metabolism, whereas ARL11 (or ARLTS1) belongs to the ARF family of the Ras superfamily of small GTPases, known to be involved in multiple regulatory pathways altered in human carcinogenesis.

Gene products are clustered (grouped) based on their Gene Ontology (GO) annotations. Genes that have similar annotations belong to the same cluster. The distance between gene products is defined using information-theoretical semantic similarity measures.

In the **Table 10** below, the most informative (most specific) common GO terms of each cluster are displayed. All gene products in the cluster are annotated with the GO term mentioned. A GO term is more informative if it occurs rarely in the GO annotations of the whole genome. The priori column contains the a priori probability p that a random gene product is annotated with the given GO term. Information is defined as log₂p. Generally, clusters with (1) a large number of gene products and (2) common GO terms with high information content are the most interesting.

**Table 10. Gene products are clustered (grouped) based on their Gene Ontology (GO) annotations.**

| CLUSTER | SIZE | GOID | PRIOR | INFO | DESCRIPTION |
|---|---|---|---|---|---|
| G1 | 4 | GO:0003824 | 0.230 | 2.120 | Catalytic activity |
| | | GO:0008152 | 0.312 | 1.681 | Metabolic process |
| | | GO:0044464 | 0.906 | 0.142 | Cell part |
| G2 | 3 | GO:0005215 | 0.046 | 4.435 | Transporter activity |
| | | GO:0006810 | 0.093 | 3.434 | Transport |
| G3 | 5 | GO:0005886 | 0.124 | 3.014 | Plasma membrane |
| | | GO:0016021 | 0.126 | 2.989 | Integral to membrane |
| G4 | 2 | GO:0005634 | 0.163 | 2.620 | Nucleus |
| | | GO:0005515 | 0.237 | 2.079 | Protein binding |
| | | GO:0050794 | 0.262 | 1.932 | Regulation of cellular process |

| | | | | | |
|---|---|---|---|---|---|
| CLUSTER MEMBERS G1 GAL3ST1 GK RDHE2 CECR1 G2 AQP10 ENST00000327625 CLCA3 G3 GNRHR OPRK1 DNER IL23R FLJ33641 G4 BCL6B FAM107A | | | | | |

In addition to the membrane cluster genes *IL23R* and *GnRHR* discussed previously, other genes in the membrane cluster (**Figure 8**) included *GNGT1, CYSLTR2,* and *SMR3B. GNGT1* codes for the gamma subunit of transducin, found mainly in rod outer segments. CYSLTR2 belongs to cysteinyl leukotrienes which are important mediators of cell trafficking and innate immune responses, involved in the pathogenesis of inflammatory processes. IFN-gamma induces CysLTR2 expression and enhances CysLT-induced inflammatory responses. *SMR3B* (submaxillary gland androgen regulated protein 3,B), is a candidate tumor related gene.

The nucleus cluster included the *FAM107A* gene, also called *TU3A*, which has been reported to be epigenetically silenced in a variety of cancers. In addition, we identified the *BCLB6* gene, a transcription factor -coding gene, upregulated by glial cell line-derived neurotrophic factor. The complete list of genes In each cluster is given in **Figure 8****.** Other genes of interest which are linked to inflammation were also found among the upregulated genes, including *Vanin3* a member of the Vanin family of secreted or membrane-associated ectoenzymes. *Vanin 1* and *Vanin 3* have recently shown to have proinflammatory activity.

When the colon and glioma cells were treated as specific groups, we identified 39 genes consistently upregulated in the *NAV3*-silenced normal colon cells and 28 genes upregulated In the glial cells (**Tables 11** and **12**) *DNER* is a transmembrane protein carrying extracellular EGF-like repeats and an atypical Notch ligand, and which was upregulated only in *NAV3* silenced colon cells (**Table 11**).

**Table 11. List of the genes upregulated in all four colon cell NAV3-silencing experiments. The total number of genes was 39, but genes lacking annotation were left out from the table. Genes are listed according to their GO-cluster. Genes marked with asterix (*) have previously been related to cancer.**

| **Abbreviation** | **Full name** | **Max fold change** | **Min fold change** |
|---|---|---|---|
| *Membrane proteins* | | | |
| GNRHR* | Homo sapiens gonadotropin-releasing hormone receptor (GNRHR), transcript valiant 1 | 32.4 | 14.8 |
| FLJ33641 | Homo sapiens hypothetical protein FL33641 (FLJ33641), RNA [NM 1526871 | 23.4 | 7.0 |
| ENST00000327625 | Unknown | 17.8 | 6.9 |
| AQP10 | Homo sapiens aquaporin 10 (AQP10) | 16.2 | 5.5 |
| IL23R | Homo sapiens interleukin 23 receptor (IL23R) | 14 0 | 4.4 |
| DNER | Homo sapiens delta-notch-like EGF repeat-containing transmembrane (DINER) | 21.9 | 5.8 |
| OPRK1 | Homo sapiens opioid receptors, kappa 1 (OPRK1) | 14.6 | 4.9 |
| GAL3ST1 | Homo sapiens galactose-3-O-Sulfotransferase 1 (GAL3ST1) | 13.2 | 3.9 |

| *Purine nucleotide binding* | | | |
|---|---|---|---|
| ARL11 | Homo sapiens ADP-ribosylation factor-like 11 (ARL11) | 30.2 | 6.8 |
| ANKRD45 | Homo sapiens ankyrin repeat domain 45 (ANKRD45), mRNA | 11.2 | 8.0 |
| GK* | Homo sapiens glycerol kinase (GK), transcript variant 1 | 15 1 | 4.1 |

| *Regulation of cellular process* | | | |
|---|---|---|---|
| FAM107A* | Homo sapiens family with sequence similarity 107, member A | 8.1 | 4.1 |
| BCL6B | Homo sapiens B-cell CLL/lymphoma 6, member B (zinc finger protein) (BCL6B) | 9.9 | 5.4 |

| *No GO-Cluster* | | | |
|---|---|---|---|
| C12orf42 | Homo sapiens chromosome 12 open reading frame 42 (C12orf42) | 47.8 | 176 |
| MFSD2* | Homo sapiens cDNA FLJ14490 fis, clone MAMMA1002886 [AK027396] | 34.9 | 9.9 |
| RDHE2 | Homo sapiens retinal short chain dehydrogenase reductase isoform 1 | 20.5 | 7.7 |
| ENST00000329949 | Homo sapiens POM121-like protein | 19.9 | 4 9 |
| ENST00000360623 | Homo sapiens clone F22H myosin-reactive immunoglobulin heavy chain variable region mRNA, partial cds | 17.2 | 5.5 |
| ZNF224 | Homo sapiens zinc finger protein 224 | 42.1 | 8.3 |
| CECR1 | Homo sapiens cat eye syndrome chromosome region, candidate 1 (CECR1) transcript variant 1 | 10.6 | 5.1 |
| TPD52L3 | Homo sapiens tumor protein D52-like 3 (TPD52L3), transcript variant 1 | 11.6 | 4.2 |
| VNN3 | Homo sapiens vanin 3 (VNN3), transcript variant 1 | 12.1 | 4.5 |
| CCDC62 | Homo sapiens coiled-coil domain containing 62 (CCDC62), transcript variant 1 | 8.8 | 4.9 |
| X92185 | H. sapiens mRNA for alu elements, [X92185] | 8.4 | 5.12 |
| CLCA3* | Homo sapiens chloride channel calcium activated, family member 3 (CLCA3) | 12.0 | 5.2 |

**Table 12. List of commonly up regulated genes in the glial cell lines.**

| **Abreviation** | **Max fold change** | **Min fold change** | **Full name** |
|---|---|---|---|
| C12orf42 | 36.9 | 12.7 | Homo sapiens chromosome 12 open reading frame 42 (C12orf42), mRNA [NM_198521] |
| AK021467 | 37.0 | 9.1 | Homo sapiens cDNA FLJ11405 fis, clone HEMBA1000769, [AK021467] |
| GNRHR | 32.3 | 4.7 | Homo sapiens gonadotropin-releasing hormone receptor (GNRHR), transcript variant 1, mRNA [NM_000406] |
| AF334588 | 28.6 | 12.8 | Homo sapiens P25 mRNA, complete cds, [AF334588] |
| A_24_P799680 | 29.3 | 6.1 | Unknown |
| ENST00000329949 | 23.6 | 6.2 | Homo sapiens POM121-like protein, mRNA (cDNA clone IMAGE 40053742), [BC112340] |
| LOC348174 | 21.7 | 7.9 | Homo sapiens cDNA FLJ38732 fis, clone KIDNE2010750, [AK096051] |
| CN480368 | 17.3 | 3.8 | UI-H-EU0-azt-m-22-0-UI,s1 NCI_CGAP_Car1 Homo sapiens cDNA clone UI-H-EU0-azt-m-22-0-UI 3', mRNA sequence [CN480368] |
| THC2378839 | 16.3 | 5.4 | CR457228 FLJ20225 {Homo sapiens}, partial (19%) [THC2378839] |
| NP297856 | 15.2 | 4.0 | GB\|AF132199,1\|AAG35545,1 PRO1460 [NP297856] |
| VNN3 | 12.4 | 4.9 | Homo sapiens vanin 3 (VNN3), transcript variant 1, mRNA [NM_018399] |
| CYSLTR2 | 12.3 | 3.5 | Homo sapiens cysteinyl leukotriene receptor 2 (CYSLTR2), mRNA [NM_020377] |
| ENST00000360623 | 16.1 | 9.6 | Homo sapiens clone F22H myosin-reactive immunoglobulin heavy chain variable region mRNA, partial cds, [AF035042] |
| THC2407334 | 15.0 | 4.2 | Unknown |
| AA903523 | 12.2 | 3.6 | AA93553 ok50h02,s1 NCI_CGAP_Lei2 Homo sapiens cDNA clone IMAGE:1517427 3', mRNA sequence [AA903523] |
| ENST00000372127 | 11.2 | 3.9 | Homo sapiens hypothetical gene supported by BC006119, mRNA (DNA clone IMAGE:3505629), partial cds, [ABC006119] |
| C19orf30 | 10.9 | 3.8 | Homo sapiens chromosome 19 open reading frame 30 (C19orf30), mRNA [NM_174947] |
| ZSCAN4 | 10.8 | 5.3 | Homo sapiens zinc finger and SCAN domain containing 4 (ZSCAN4), mRNA [NM_152677] |
| SPAG11 | 10.8 | 5.3 | Homo sapiens sperm associated antigen 11 (SPAG11), transcript variant C, mRNA [NM_058203] |
| A_24_P922440 | 10.3 | 4.9 | Unknown |
| THC2443880 | 9.7 | 4.2 | Unknown |
| UNQ6490 | 19.4 | 5.5 | Homo sapiens clone DNA147309 YPLR6490 (UNQ6490) MRNA, complete cds, [AY358209] |
| ARL11 | 18.1 | 7.6 | Homo sapiens ADP-ribosylation factor-like 11 (ARL11), mRNA [NM 138450] |
| THC2314833 | 7.2 | 3.9 | Unknown |
| IL23R | 8.7 | 3.4 | Homo sapiens interleukin 23 receptor (IL23R), mRNA [NM 144701] |
| FLJ33641 | 9.8 | 5.9 | Homo sapiens hypothetical protein FLJ33641 (FLJ33641), mRNA [NM 152687] |
| U22172 | 7.0 | 4.6 | Human DNA damage repair and recombination protein RAD52 pseudogene mRNA, partial cds, [U22172] |
| BX113452 | 14.7 | 6.0 | BX113452 Soares infant brain 1NIB Homo sapiens cDNA clone IMAGp998L21165, mRNA sequence [BX113452] |

### Example 5. immunohistochemistry

### IL23R and beta-catenin immunohistochemistry on tissue samples

IL23R and beta-catenin expression were studied by immunohisto-chemistry in tissue microarrays prepared from paraffin-embedded colon biop-sies. From each patient, paired samples from the histologically normal colon and two samples from the colon tumour were included in the array. Also, from 10 patients paired samples of an adenomatous lesion and another sample from the normal colon, were included. Altogether 57 patients, 43 MSS tumour samples, 14 MSI tumour samples, 14 adenoma samples and 57 corresponding normal colon samples were Included in the tissue microarrays.

Immunohistochemistry was carried out by using the avidin-biotin-peroxidase complex tehnique (Vectastain Elite ABC kit [mouse IgG], Vector laboratories, Burlingame, CA, USA) with DAB as chromogenic substrate and Mayer's hematoxylin as counterstain. Following standard deparaffinization, endogenous peroxidase activity was blocked in 3% H₂O₂ in PBS for 10 min and the sections were pretreated in a +95°C water bath in citrate buffer (DakoCy-tomation, Glostrup, Denmark) for 20 min. The slides were then incubated with mouse monoclonal antibodies against IL23R (R&D MAB14001; diluted in 1:30, slides pretreated with 1% trypsin at 37°C 30 min) or against beta-catenin (Zymed CAT-5H10, Invitrogen, Carlsbad, CA 92008 U.S.A; diluted in 1:250, slide pretreatment at +95°C for 30 min in DAKO Target Retrieval Solution S1699, pH 6-6,2) at +4°C overnight. DAB was used as colourigenic substrate for IL23R while VECTOR NovaRED substrate kit S-4800 was used for beta-catenin.

For statistical analyses, the immunolabelling result was scored as follows: for IL23R immunoreactivity no staining (score 0), weak positive staining (score 1), clearly positive staining (score 2), strongly positive staining (score 3; see also **Figure 9** for examples) for beta-catenin no staining (score 0), cell membrane staining (score 1), cytoplasmic staining (score 2), nuclear staining in the majority of tumour cells (score 3; see also **Figure 9**).

### Statistical analyses

Correlations between categorical variables were analyzed using the chi-square test, or when not valid, the Fisher's exact test. Survival analyses were performed using death caused by colon cancer as the primary endpoint. Follow-up times were obtained from Statistics Finland, a government agency. SPSS version 15.0 software (SPSS, IL, USA) was used.

### Expression of IL23R or nuclear beta-catenin in NAV3 aberrant MSS tumors associates with Dukes staging and lymph node metastases, and expression of IL23R associates with poor prognosis

Immunohistochemital detection of beta-catenin (linked to the GNRHR pathway) and IL23R expression were then performed on a tissue microarray including 43 MSS (including also 8 adenoma samples of the same patients) and 14 MSI patient samples (tumour and corresponding normal colon epithelium). In all normal colon samples of these cases, the beta-catenin staining was always membranous and no or only weak IL23R expression (grade 1) was found except for three cases (**Figure 9**).

In normal epithelial cells beta-catenin is known to locate in cell membranes but changes its distribution toward the nucleus and cytoplasm in carcinoma samples. In the representative MSS tumour samples (duplicate samples of each), nuclear beta-catenin was found in 10/43 cases and upregulation of IL23R-immunoreactivity (scores 2-3) in 27/43 cases. The nuclear beta-catenin expression correlated with lymph node metastases. Up-regulated IL23R-immunoreactivity in tissue array samples strongly correlated with Dukes staging (see **Table 3** in Example 2, NAV3 aberrations associate with chromosome 12 polysomy and with lymph node metastasis) and also with lymph node metastases (see **Table 3** in Example 2). Tumor samples with allelic NAV3 deletions showed most frequently clearly up-regulated IL23R reactivity (**Figure 9**).

In a Kaplan-Meier survival analysis (**Figure 10****),** patients with up-regulated IL-23R immunoreactivity (scores 2 and 3) were found to have a worse prognosis than patients with normal or low expression level (i.e. immunohistological staining intensity, scored 0 or 1, respectively, and comparable to that of the normal colon samples) (see **Table 3** in Example 2). The mean survival time for patient with low IL23R expression was 44 months (95%Cl 38-50 moths), whereas for patient with up-regulated high expression, the mean survival was only 23 months (95%Cl 13-33 moths).

For 11 MSS type CRC patients, it was possible to compare the IL23R, beta-catenin and *NAV3* FISH results in the adenoma and tumour samples, *NAV3* copy number changes, together with elevated IL23R expression (moderate or strong immunostaining) and/or nuclear beta-catenin were present in 3 of the adenoma samples and elevated IL23R expression alone in two additional adenomas (data not shown), In the corresponding tumour samples, the finding was similar except for two samples where no *NAV3* aberration was found despite the upregulated IL23R expression. In both of these latter cases, the corresponding adenoma lesion had shown chromosome 12 polysomy, however.

### IL23R and GnRHR Immunohistochemistry on tissue samples

GnRHR immunostaining was performed in a LabVision immunostainer (Labvision, CA, USA) following antigen retrieval using Tris-EDTA buffer, pH 9.0 in a microwave oven for 24 minutes at 900 watts followed by cooling for 20 minutes at room temperature. GnRHR was detected with mouse monoclonal antibody (diluted 1:10, Abcam, Cambridge, UK) and a polymer based detection system (Envision, K5007, DakoCytomation) with diaminobenzidine (DAB) as the chromogen (**Figure 11**).

Two representative cases of colorectal tissue sections (paired samples of histologically normal colon and the colon tumor) and two cases of astrocytoma (glioblastoma) prepared from paraffin-embedded colon biopsies of patients with colorectal cancer or astrocytoma were included. For these samples, immunohistochemistry was carried out as previously described using the anti-GnRHR antibody and mouse monoclonal antibody against IL23R (R&D MAB14001; diluted 1:30, slides pretreated with 1% trypsin at 37 °C for 30 min) and avidin-biotin-peroxidase complex detection (Vectastain Elite ABC kit [mouse IgG], Vector Laboratories, Burlingame, CA, USA) with DAB as the chromogenic substrate.

### Confirmation of IL23 and GnRHR protein upregulation in NAV3 silenced 0205 cells, and CRC and glioblastoma tissue samples

Upregulation of the GnRHR protein levels in *NAV3*-silenced 0205 cells was demonstrated by immunostaining. GnRHR staining was higher in *NAV3*-silenced cells than wild type cells and siRNA scrambled control transfected cells (**Figure 11 a-c**). GnRHR protein immunostaining in CRC tissue samples was also higher than in normal colon epithelium (**Figure 11** **d**). Results were repeatable in two typical CRC patient samples, one case with a MSS type CRC and 41 % of *NAV3*-deleted cells in the tumor (cut-off for *NAV3* deletion is 3% in normal reference samples as reported earlier, Figure 11 e), and one case of MSI type CRC and 8% of *NAV3*-deleted cells in the tumor (**Figure 11** **f**). Likewise, the upregulation of GNRHR and IL23R was observed in a preliminary series of glioblastoma (astrocytoma) samples showing NAV3 deletion compared to glial tumors with no deletion (**Figure 11 g and h**).

## Claims

1. A method of demonstrating the malignant character of a tumor or cell subpopulation in a subject, the method comprising:
i) determining a NAV3 copy number change in a biological sample from the subject; and
ii) determining overexpression of at least one gene or gene product selected from IL23R, GnRHR, beta-catenin, in the biological sample or in another biological sample from the subject;
iii) demonstrating the malignant character of a tumor or cell subpopulation in a subject, when both a NAV3 copy number change and over-expression of at least one gene or gene product selected from IL23R, GnRHR, beta-catenin are present in the sample(s) from the subject

2. A method of predicting a prognosis of a subject having a tumor, the method comprising:
i) determining a NAV3 copy number change in a biological sample from a subject;
ii) determining overexpression of at least one gene or gene product selected from IL23R or beta-catenin, in the biological sample or in another biological sample from the subject; and
iii) predicting a prognosis of a subject having both a NAV3 copy number change and overexpression of at least one gene or gene product selected from IL23R or beta-catenin in the sample(s).

3. A use of NAV3 gene or gene product and at least one gene and/or gene product selected from IL23R, GnRHR, beta-catenin for demonstrating the malignant character of a tumor or cell subpopulation with a NAV3 copy number change and with overexpression of at least one gene or gene product selected from IL23R, GnRHR, beta-catenin, in a subject.

4. A use of NAV3 gene or gene product and at least one gene and/or gene product selected from IL23R or beta-catenin for predicting a prognosis of a subject.

5. The method or use according to any one of the previous claims, wherein the NAV3 copy number change is caused by a deletion, amplification or translocation of NAV3 gene or a major part of it.

6. The method or use according to any one of the previous claims, wherein the tumor is a colorectal tumor, brain tumor or tumor of epidermal keratinocytes.

7. The method or use according to any one of the previous claims, wherein the tumor is a carcinoma.

8. The method or use according to any one of the previous claims, wherein the gene(s) and/or gene product(s) is activated, inactivated, overexpressed or underexpressed, or amount of the gene product is increased or decreased.

9. The method or use according to any one of claims 1, 3 or 5-8, wherein a presence of a tumor with a NAV3 copy number change and with overexpression of at least one gene or gene product selected from IL23R, GnRHR, beta-catenin is associated with lymph node metastases, high grade malignancy and/or poor survival.

10. The method or use according to any one of claims 2, 4 or 5-8, wherein a presence of a tumor with a Nav3 copy number change and with over expression of at least one gene or gene product selected from IL23R or beta-catenin is associated with lymph node metastases, high grade malignancy and/or poor survival.

11. The method or use according to any one of claims 1, 3 or 5-9, wherein the gene(s) selected from IL23R, GnRHR, beta-catenin, codes for a protein, which is a membrane protein, a protein regulating cellular processes, or a purine nucleotide binding protein.

12. The method or use according to anyone of claims 2, 4, 5-8 or 10, wherein the gene(s) selected from IL23R or beta-catenin, codes for a protein, which is a membrane protein, a protein regulating cellular processes, or a purine nucleotide binding protein.

13. The method or use according to any one of claims 1, 3 or 5-9 or 11, wherein the gene or gene product is IL23R or/and GnRHR.

14. The method or use according to any one of the previous claims, wherein the brain tumor is a glioma.

## Patentansprüche

1. Verfahren des Aufzeigens des malignen Charakters eines Tumors oder einer Zelluntergruppe in einem Individuum, wobei das Verfahren Folgendes umfasst:
i) Feststellen einer Veränderung der NAV3-Kopienzahl in einer biologischen Probe des Individuums; und
ii) Feststellen der Überexpression mindestens eines Gens oder Genprodukts, ausgewählt aus IL23R, GnRHR, Beta-Catenin, in der biologischen Probe oder in einer anderen biologischen Probe von dem Individuum;
iii) Aufzeigen des malignen Charakters eines Tumors oder einer Zelluntergruppe in einem Individuum, wenn sowohl eine Veränderung der NAV3-Kopienzahl als auch eine Überexpression mindestens eines Gens oder Genprodukts, ausgewählt aus IL23R, GnRHR, Beta-Catenin, in der Probe bzw. in den Proben des Individuums vorhanden sind.

2. Verfahren des Vorhersagens einer Prognose des Aufweisens eines Tumors für ein Individuum, wobei das Verfahren Folgendes umfasst:
i) Feststellen einer Veränderung der NAV3-Kopienzahl in einer biologischen Probe des Individuums;
ii) Feststellen der Überexpression mindestens eines Gens oder Genprodukts, ausgewählt aus IL23R oder Beta-Catenin, in der biologischen Probe oder in einer anderen biologischen Probe von dem Individuum; und
iii) Vorhersagen einer Prognose des Aufweisens sowohl einer Veränderung der NAV3-Kopienzahl als auch einer Überexpression mindestens eines Gens oder Genprodukts, ausgewählt aus IL23R oder Beta-Catenin, in der Probe bzw. in den Proben für ein Individuum.

3. Verwendung des NAV3-Gens oder -Genprodukts und mindestens eines Gens bzw. Genprodukts, ausgewählt aus IL23R, GnRHR, Beta-Catenin, zum Aufzeigen des malignen Charakters eines Tumors oder einer Zelluntergruppe mit einer Veränderung der NAV3-Kopienzahl und Überexpression mindestens eines Gens oder Genprodukts, ausgewählt aus IL23R, GnRHR, Beta-Catenin, in einem Individuum.

4. Verwendung des NAV3-Gens oder -Genprodukts und mindestens eines Gens bzw. Genprodukts, ausgewählt aus IL23R oder Beta-Catenin, zum Vorhersagen einer Prognose für ein Individuum.

5. Verfahren oder Verwendung nach einem der vorhergehenden Ansprüche, wobei die Veränderung der NAV3-Kopienzahl durch eine Deletion, Amplifikation oder Translokation des NAV3-Gens oder eines wesentlichen Teils davon verursacht wird.

6. Verfahren oder Verwendung nach einem der vorhergehenden Ansprüche, wobei der Tumor ein Kolorektaltumor, ein Gehirntumor oder ein Tumor epidermaler Keratinozyten ist.

7. Verfahren oder Verwendung nach einem der vorhergehenden Ansprüche, wobei der Tumor ein Karzinom ist.

8. Verfahren oder Verwendung nach einem der vorhergehenden Ansprüche, wobei das Gen bzw. die Gene bzw. das Genprodukt bzw. die Genprodukte aktiviert, inaktiviert, überexprimiert oder unterexprimiert ist bzw. sind oder die Menge des Genprodukts erhöht oder verringert ist.

9. Verfahren oder Verwendung nach einem der Ansprüche 1, 3 oder 5-8, wobei ein Vorhandensein eines Tumors mit einer Veränderung der NAV3-Kopienzahl und mit Überexpression mindestens eines Gens oder Genprodukts, ausgewählt aus IL23R, GnRHR, Beta-Catenin, mit Lymphknotenmetastasen, hochgradiger Malignität bzw. ungünstigem Überleben assoziiert ist.

10. Verfahren oder Verwendung nach einem der Ansprüche 2, 4 oder 5-8, wobei ein Vorhandensein eines Tumors mit einer Veränderung der NAV3-Kopienzahl und mit Überexpression mindestens eines Gens oder Genprodukts, ausgewählt aus IL23R oder Beta-Catenin, mit Lymphknotenmetastasen, hochgradiger Malignität bzw. ungünstigem Überleben assoziiert ist.

11. Verfahren oder Verwendung nach einem der Ansprüche 1, 3 oder 5-9, wobei das Gen bzw. die Gene, ausgewählt aus IL23R, GnRHR, Beta-Catenin, ein Protein kodiert bzw. kodieren, das ein Membranprotein, ein Protein, das zelluläre Prozesse reguliert, oder ein Purinnukleotid-Bindungsprotein ist.

12. Verfahren oder Verwendung nach einem der Ansprüche 2, 4, 5-8 oder 10, wobei das Gen bzw. die Gene, ausgewählt aus IL23R oder Beta-Catenin, ein Protein kodiert bzw. kodieren, das ein Membranprotein, ein Protein, das zelluläre Prozesse reguliert, oder ein Purinnukleotid-Bindungsprotein ist.

13. Verfahren oder Verwendung nach einem der Ansprüche 1, 3 oder 5-9 oder 11, wobei das Gen oder Genprodukt IL23R bzw. GnRHR ist.

14. Verfahren oder Verwendung nach einem der vorhergehenden Ansprüche, wobei der Gehirntumor ein Gliom ist.

## Revendications

1. Procédé pour démontrer le caractère malin d'une tumeur ou d'une sous-population cellulaire chez un sujet, le procédé comprenant :
i) la détermination d'un changement du nombre de copies de NAV3 dans un échantillon biologique du sujet ; et
ii) la détermination de la surexpression d'au moins un gène ou produit génique choisi parmi IL23R, GnRHR, bêta-caténine dans l'échantillon biologique ou dans un autre échantillon biologique du sujet ;
iii) la démonstration du caractère malin d'une tumeur ou d'une sous-population cellulaire chez un sujet, lorsque à la fois un changement du nombre de copies de NAV3 et la surexpression d'au moins un gène ou produit génique choisi parmi IL23R, GnRHR, bêta-caténine sont présents dans l'échantillon ou les échantillons du sujet.

2. Procédé pour prédire un pronostic d'un sujet ayant une tumeur, le procédé comprenant :
i) la détermination d'un changement du nombre de copies de NAV3 dans un échantillon biologique d'un sujet ;
ii) la détermination de la surexpression d'au moins un gène ou produit génique choisi parmi IL23R ou bêta-caténine dans l'échantillon biologique ou dans un autre échantillon biologique du sujet ;
iii) la prédiction d'un pronostic d'un sujet ayant à la fois un changement du nombre de copies de NAV3 et la surexpression d'au moins un gène ou produit génique choisi parmi IL23R ou bêta-caténine dans l'échantillon ou les échantillons.

3. Utilisation d'un gène ou produit génique de NAV3 et d'au moins un gène et/ou produit génique choisi parmi IL23R, GnRHR, bêta-caténine pour démontrer le caractère malin d'une tumeur ou d'une sous-population cellulaire avec un changement du nombre de copies de NAV3 et avec la surexpression d'au moins un gène ou produit génique choisi parmi IL23R, GnRHR, bêta-caténine chez un sujet.

4. Utilisation d'un gène ou produit génique de NAV3 et d'au moins un gène et/ou produit génique choisi parmi IL23R ou bêta-caténine pour prédire un pronostic d'un sujet.

5. Procédé ou utilisation selon l'une quelconque des revendications précédentes, dans lequel/laquelle le changement du nombre de copies de NAV3 est provoqué par une délétion, une amplification ou une translocation du gène de NAV3 ou d'une partie majeure de celui-ci.

6. Procédé ou utilisation selon l'une quelconque des revendications précédentes, dans lequel/laquelle la tumeur est une tumeur colorectale, une tumeur du cerveau ou une tumeur de kératinocytes épidermiques.

7. Procédé ou utilisation selon l'une quelconque des revendications précédentes, dans lequel/laquelle la tumeur est un carcinome.

8. Procédé ou utilisation selon l'une quelconque des revendications précédentes, dans lequel/laquelle le(s) gène(s) et/ou produit(s) génique(s) est/sont activé(s), inactivé(s), surexprimé(s) ou sous-exprimé(s) ou bien la quantité du produit génique est augmentée ou diminuée.

9. Procédé ou utilisation selon l'une quelconque des revendications 1, 3 ou 5 à 8, dans lequel/laquelle une présence d'une tumeur avec un changement du nombre de copies de NAV3 et avec la surexpression d'au moins un gène ou produit génique choisi parmi IL23R, GnRHR, bêta-caténine est associée à des métastases de noeuds lymphatiques, une malignité de niveau élevé et/ou une survie médiocre.

10. Procédé ou utilisation selon l'une quelconque des revendications 2, 4 ou 5 à 8, dans lequel/laquelle une présence d'une tumeur avec un changement du nombre de copies de NAV3 et avec la surexpression d'au moins un gène ou produit génique choisi parmi IL23R ou bêta-caténine est associée à des métastases de noeuds lymphatiques, une malignité de niveau élevé et/ou une survie médiocre.

11. Procédé ou utilisation selon l'une quelconque des revendications 1, 3 ou 5 à 9, dans lequel/laquelle le(s) gène(s) choisi(s) parmi IL23R, GnRHR, bêta-caténine code(nt) une protéine, qui est une protéine membranaire, une protéine régulant des processus cellulaires, ou une protéine se liant aux nucléotides puriniques.

12. Procédé ou utilisation selon l'une quelconque des revendications 2, 4, ou 5 à 8 ou 10, dans lequel/laquelle le(s) gène(s) choisi(s) parmi IL23R ou bêta-caténine code(nt) une protéine, qui est une protéine membranaire, une protéine régulant des processus cellulaires, ou une protéine se liant aux nucléotides puriniques.

13. Procédé ou utilisation selon l'une quelconque des revendications 1, 3 ou 5 à 9 ou 11, dans lequel/laquelle le gène ou produit génique est IL23R et/ou GnRHR.

14. Procédé ou utilisation selon l'une quelconque des revendications précédentes, dans lequel/laquelle la tumeur du cerveau est un gliome.
